# EUROPEAN PATENT APPLICATION

(11) **EP 4 434 538 A1**
(43) Date of publication of application: **25.09.2024**
(21) Application number: 23162925.4
(22) Date of filing: 20.03.2023
(51) Int. Cl.: A61K 38/48, A61K 38/49, A61P 25/00, A61P 25/22, A61P 25/24, A61P 25/30, G01N 33/50, A61K 31/00

(54) **METHODS OF PREVENTING OR TREATING PSYCHIATRIC DISEASES THROUGH MODULATION OF THE PLASMINOGEN-PLASMIN CASCADE**

(71) Applicant: Koch, Philipp, 68309 Mannheim (DE); Schmidt, Malin, 76327 Pfinztal (DE)
(72) Inventor: Koch, Philipp, 68309 Mannheim (DE); Schmidt, Malin, 76327 Pfinztal (DE)
(74) Representative: Krauss, Jan

(57) **Abstract**

The present invention relates to at least one of a protein selected from group consisting of uPA, tPA, plasminogen, plasmin, and functional fragments thereof, or a nucleic acid encoding said uPA, tPA, plasminogen, plasmin, and functional fragments thereof for use in the prevention or treatment of a psychiatric disorder or disease in a subject. The present invention further relates to a method for identifying a compound that increases the expression, amount and/or biological activity of a protein of the AIIt complex and/or PLGRKT in a cell of a subject, in particular a neuronal cell, such as, for example, a human induced pluripotent stem cell-derived neuronal cell. Further provides are pharmaceutical compositions for use in medicine, in particular for use in the prevention or treatment of a psychiatric disorder or disease in a subject. Further provided are methods for improving or amplifying the therapeutic effect of electroconvulsive therapy (ECT), psychotherapy or a compound in the prevention or treatment of a psychiatric disorder or disease in a subject. Preferably the improvement or amplification of the therapeutic effect is synergistic.

## Description

The present invention relates to at least one of a protein selected from group consisting of uPA, tPA, plasminogen, plasmin, and functional fragments thereof, or a nucleic acid encoding said uPA, tPA, plasminogen, plasmin, and functional fragments thereof for use in the prevention or treatment of a psychiatric disorder or disease in a subject. The present invention further relates to a method for identifying a compound that increases the expression, amount and/or biological activity of a protein of the AIIt complex and/or PLGRKT in a cell of a subject, in particular a neuronal cell, such as, for example, a human induced pluripotent stem cell-derived neuronal cell. Further provides are pharmaceutical compositions for use in medicine, in particular for use in the prevention or treatment of a psychiatric disorder or disease in a subject. Further provided are methods for improving or amplifying the therapeutic effect of electroconvulsive therapy (ECT), psychotherapy or a compound in the prevention or treatment of a psychiatric disorder or disease in a subject. Preferably the improvement or amplification of the therapeutic effect is synergistic.

### Background of the invention

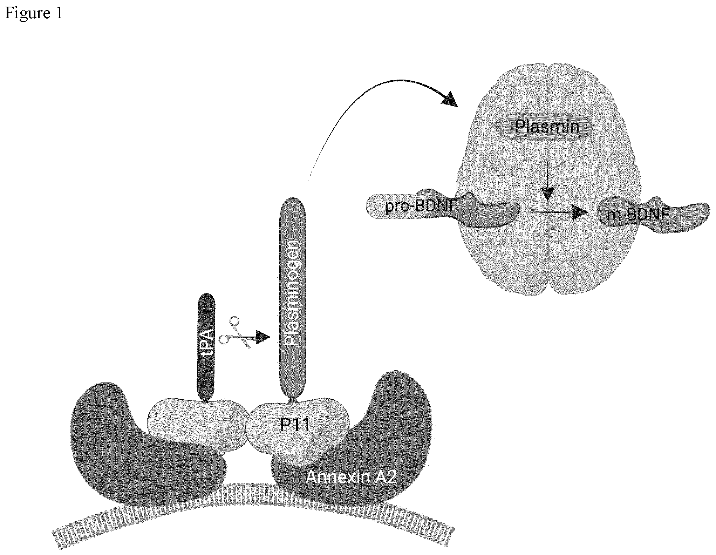

m-BDNF (*mature brain derived neurotrophic factor*) is a neurotrophic factor that is synthesized by the neuronal cell in the form of the precursor pro-BDNF. The final factor is then generated after cleaving off the pro-domain thereof¹. Pro-BDNF and m-BDNF have different binding partners and have opposing biological functions². While pro-BDNF triggers apoptotic cellular processes, m-BDNF modulates the synaptic plasticity of the neuronal cells². There are several enzymes that can convert pro-BDNF into m-BDNF. One of these is plasmin, which cleaves pro-BDNF into m-BDNF mainly in the extracellular space of the brain (mediated by tPA, *tissue plasminogen activator*)³*.* Plasmin is processed from the precursor plasminogen. During this process, plasminogen binds to the AIIt complex (a tetrameric complex that is essentially composed of two copies of annexin A2 proteins and one S100A10, alias p11, dimer). In addition, tPA binds to this complex, and uPA is involved. These enzymes cleave plasminogen into plasmin (see Figure 1). This complex is important also in other regions of the body, and thus essentially provides the function of fibrinolysis in the body (for a summary, see, for example,⁴).

It was assumed that a distorted ratio of pro-BDNF and m-BDNF that is due to an incorrect or reduced processing could be involved in the etiology of psychiatric diseases^{5,6}. pro-BDNF levels are increased in depressive patients, whereas the levels of m-BDNF are reduced, when compared to healthy controls⁵⁻¹². One study showed that, in an animal model, chronic stress led to a depressive phenotype that was associated with an increase of the pro-BDNF level and a reduction of the m-BDNF level⁹. The ratio of pro-BDNF to m-BDNF was increased in the hippocampus⁹. This disbalance could be related to reduced neuroplasticity¹². At the same time, the administration of pro-BDNF led to a depressive phenotype and reduced neuroplasticity that later could be reverted through the external administration of BDNF¹². Some anti-depression medicaments reversed an elevated pro-BDNF level and more reduced tPA and BDNF-levels, as well as a reduced BDNF/pro-BDNF ratio in depressive patients⁵.

The above publications concluded that the balance between the pro-BDNF/p75NTR/sortilin and m-BDNF/TrkB signaling pathways appears to be dysregulated in major depression, and that both pathways could be considered as biomarkers for major depression⁷. They demonstrated that levels of p11 are regulated in depression and by antidepressant regimens and, conversely, that p11 regulates depression-like behaviors and/or responses to antidepressants. Therefore, current, and future studies of p11 are thought to provide the molecular and cellular framework for a development of novel antidepressant therapies¹³.

US8470548B2 discloses a biological association between p11 and 5-HT1B and 5-HT4 receptors, providing the use of p11 as a drug target for diseases involving these receptors, as well as a diagnostic tool for the identification of patients suffering from p11/5-HT receptor related disorders. The patent claims a method of diagnosing depression in a subject comprising determining the level of p11 in a biological sample wherein said biological sample comprises brain tissue or peripheral blood mononuclear cells from said subject and comparing said p11 level to a reference, wherein a reduced level of p11 compared to the reference constitutes a positive diagnosis of depression.

Idell RD, et al.¹⁴ disclose that tissue plasminogen activator (tPA) is a key factor that not only promotes fibrinolysis via the activation of plasminogen, but also contributes to regulation of synaptic plasticity and neurogenesis through plasmin-mediated activation of a probrain derived neurotrophic factor (BDNF) to mature BDNF. Pro-BDNF activation could potentially be suppressed by competition with fibrin for plasmin and tPA. High affinity binding of plasmin and tPA to fibrin could result in a decrease of pro-BDNF activation during brain inflammation leading to fibrosis further perpetuating depressed mood. They propose that within the brain, an imbalance between tPA and urokinase plasminogen activator (uPA) and plasminogen activator inhibitor-1 (PAI-1) and neuroserpin favors the inhibitors, resulting in changes in neurogenesis, synaptic plasticity, and neuroinflammation that result in depressive behavior. They hypothesize that psychedelic 5-HT2A receptor agonists, such as psilocybin, can improve mood through antiinflammatory and pro-fibrinolytic effects that include blockade of TNF-α activity leading to decreased PAI-1 activity and increased clearance. The process involves disinhibition of tPA and uPA with subsequent increased cleavage of pro-BDNF which promotes neurogenesis, decreased neuroinflammation, decreased fibrin deposition, normalized glial-neuronal crosstalk, and optimally functioning neuro-circuits involved in mood.

The use of 5-HT2A receptor agonists for a treatment of depression is known. Nevertheless, ECT, psychopharmaceuticals (such as antidepressive agents), ketamine and/or 5-HT2A receptor agonists or other treatment forms, depending on the individual patient, show a good or lesser response without that this can currently be influenced. The long-term use of antidepressive agents (including the novel SSRI) causes side-effects, there may be a delay of onset, a recurrency of symptoms, a possible dependency on environmental factors, and/or an antidepressant tolerance (tachyphylaxis), and ECT can, for example, lead to short-term amnesia. Many patients show a low response, and therefore often several antidepressive agents are combined. In addition, there is a large number of patients that suffer from so-called "treatment-resistant depression". In these cases, regular therapies do not work, and novel treatment strategies are urgently required¹⁵⁻²¹.

New and improved strategies are therefore sought in order to prevent or treat psychiatric diseases, such as depression, and to improve neuroplasticity. It is therefore an object of the present invention to provide such strategies. Other objects and advantages will readily become apparent for the person of skill from studying the following more detailed description and examples.

In a first aspect of the present invention, the problem of the present invention is solved by providing at least one of a protein selected from group consisting of tPA, uPA, plasminogen, plasmin, and functional fragments thereof, or a nucleic acid encoding said tPA, uPA, plasminogen, plasmin, and functional fragments thereof for use in the prevention or treatment of a psychiatric disorder or disease in a subject. Preferred is the at least one protein for use according to the present invention, wherein the protein tPA, uPA and plasminogen or functional fragments thereof, or a nucleic acid encoding said tPA, uPA and plasminogen or functional fragments thereof are used in combination.

In a second aspect of the present invention, the problem of the present invention is solved by providing a method for identifying a compound that increases the expression, amount and/or biological activity of a protein of the AIIt complex and/or PLGRKT in a cell of a subject, comprising the steps of i) contacting at least one candidate compound with a cell that expresses at least one protein of the AIIt complex and/or PLGRKT, and ii) detecting an increase of the expression, amount and/or biological activity of the at least one protein of the AIIt complex and/or PLGRKT in the cell in the presence of the candidate compound compared to the expression, amount and/or biological activity in the absence of the candidate compound, optionally in the presence of at least one 5-HT2A receptor agonist, ketamine, SSRI antidepressant, mGluR2 agonist, mGluR2/3 agonist, allosteric modulator (e.g. PAM), cytokine, and/or ECT, wherein an increase of the expression, amount and/or biological activity of the at least one protein of the AIIt complex and/or PLGRKT in the cell identifies a compound that increases the expression, amount and/or biological activity of a protein of the AIIt complex and/or PLGRKT in a cell of a subject.

In a third aspect of the present invention, the problem of the present invention is solved by providing a method for producing a pharmaceutical composition, comprising performing a method according to the present invention as above, and admixing the compound as identified with at least one pharmaceutically carrier. Further provided is a pharmaceutical composition, comprising at least one protein or functional fragment thereof, at least one nucleic acid or functional fragment thereof, at least one expression cassette or at least one vector for use according to the present invention, together with at least one pharmaceutically acceptable carrier, or as produced according to the present invention, for use in medicine, in particular for use in the prevention or treatment of a psychiatric disorder or disease in a subject. Preferred is the pharmaceutical composition for use according to the present invention, wherein the composition is for injection, oral and/or nasal application.

Further preferred is the pharmaceutical composition for use according to the present invention, wherein the use is for an improvement or amplification of the therapeutic effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent in the prevention or treatment of a psychiatric disorder or disease in a subject, or wherein the use is for an improvement or amplification of the therapeutic effect of electroconvulsive therapy (ECT) and/or psychotherapy in a subject.

Even further preferred is the pharmaceutical composition for use according to the present invention, wherein the use is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.

The psychiatric disorder or disease in the subject may be selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

In a fourth aspect of the present invention, the problem of the present invention is solved by providing the use of at least one of a protein selected from group consisting of tPA, uPA, plasminogen, plasmin, and functional fragments thereof, or a nucleic acid encoding said tPA, uPA, plasminogen, plasmin, and functional fragments thereof for improving or amplifying the therapeutic effect of electroconvulsive therapy (ECT), psychotherapy or a compound in the prevention or treatment of a psychiatric disorder or disease in a subject. Preferred is the use according to the present invention, wherein the protein tPA, uPA and plasminogen or functional fragments thereof, or the nucleic acid encoding said tPA, uPA and plasminogen or functional fragments thereof are used in combination.

In a fifth aspect of the present invention, the problem of the present invention is solved by providing a method for improving or amplifying the therapeutic effect of electroconvulsive therapy (ECT), psychotherapy or a compound in the prevention or treatment of a psychiatric disorder or disease in a subject comprising administering an effective amount of at least one of a protein selected from group consisting of tPA, uPA, plasminogen, plasmin, and functional fragments thereof, or a nucleic acid encoding said tPA, uPA, plasminogen, plasmin, and functional fragments thereof. Preferred is the method according to the present invention, wherein the protein tPA, uPA and plasminogen or functional fragments thereof, or the nucleic acid encoding said tPA, uPA and plasminogen or functional fragments thereof are administered in combination.

In a sixth aspect of the present invention, the problem of the present invention is solved by providing a method for preventing or treating a psychiatric disorder or disease in a subject in need of said prevention or treatment, comprising administering to the subject an effective amount of at least one of a protein selected from group consisting of tPA, uPA, plasminogen, plasmin, and functional fragments thereof, or a nucleic acid encoding said tPA, uPA, plasminogen, plasmin, and functional fragments thereof. Preferred is the method according to the present invention, wherein the protein tPA, uPA and plasminogen or functional fragments thereof, or the nucleic acid encoding said tPA, uPA and plasminogen or functional fragments thereof are administered in combination.

Preferred is the method according to the present invention, wherein the at least one protein or functional fragment thereof, at least one nucleic acid or functional fragment thereof, the at least one expression cassette or at least one vector are administered as a pharmaceutical composition together with at least one pharmaceutically acceptable carrier, or as a pharmaceutical composition produced according to the present invention. Preferred is the method according to the present invention, wherein the composition is administered by injection, oral and/or nasal application.

Further preferred is the method according to the present invention, wherein the prevention or treatment comprises an improvement or amplification of the effect of a compound selected from the group consisting of an 5-HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent or an improvement or amplification of the therapeutic effect of electroconvulsive therapy (ECT) and/or psychotherapy in the subject. The treatment can be as an adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.

Further preferred is the method according to the present invention, wherein the psychiatric disorder or disease in the subject is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

As mentioned above, in a first aspect of the present invention, the object of the present invention is solved by providing at least one of a protein selected from group consisting of tPA, uPA, plasminogen, plasmin, and functional fragments thereof, or a nucleic acid encoding said tPA, uPA, plasminogen, plasmin, and functional fragments thereof for use in the prevention or treatment of a psychiatric disorder or disease in a subject. Preferred is the at least one protein for use according to the present invention, wherein the protein tPA, uPA and plasminogen or functional fragments thereof, or a nucleic acid encoding said tPA, uPA and plasminogen or functional fragments thereof are used in combination.

A manipulation of BDNF as therapeutic concept is discussed since years in the treatment of psychiatric diseases³⁰. In several therapy forms, including antidepressive agents, ECT and the treatment using ketamine, it was shown that these upregulate BDNF, p11 and tPA^{5,8,13-15,31-44}. Jointly, this can modify the ratio of pro-BDNF to m-BDNF via the plasminogen-plasmin cascade.

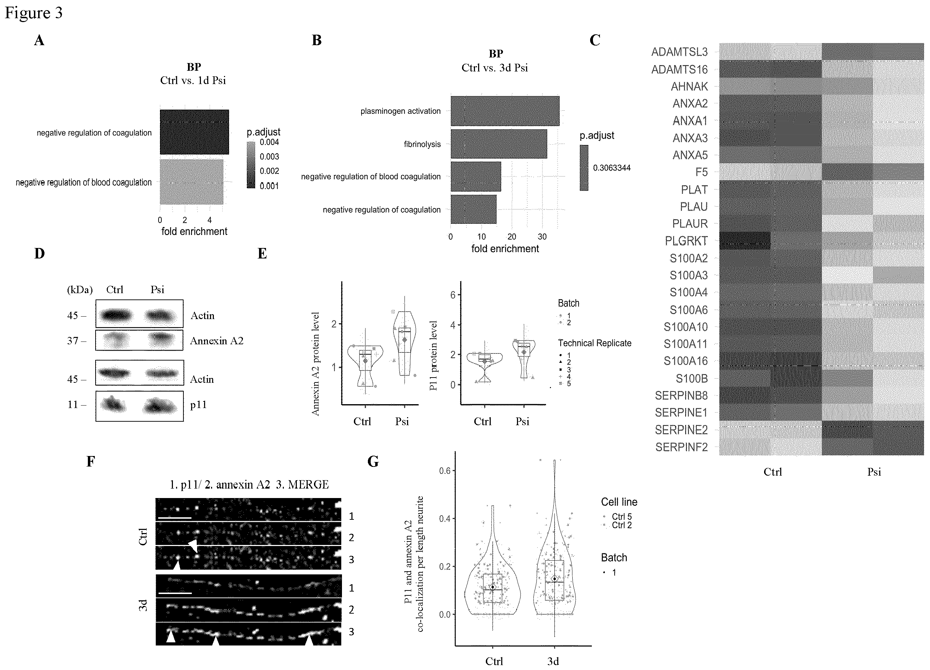

So far, plasminogen and/or tPA and/or plasmin and/or uPA themselves have not been postulated as therapeutics for a treatment of psychiatric diseases. In addition, the present results show that 5-HT2A receptor agonists, which represent a novel therapy concept, not only activate BDNF and tPA and uPA, but apparently all of the components of the AIIt complex and/or PLGRKT. For example, Figure 3C (PLAU gene and PLAUR gene, respectively) shows an upregulation of uPA and uPAR after treatment with psilocin. Figure 3C (PLGRKT gene) further shows an upregulation of the plasminogen receptor after treatment with psilocin. The available amount of the substrate plasminogen or tPA or uPA has a direct effect on the availability of plasmin and thus on the ratio of pro-BDNF to m-BDNF. An increase in the bioavailability of the core enzyme plasmin also leads to increased processing of pro-BDNF into m-BDNF. This also represents a new combinatorial measure to enhance the effectiveness of therapeutic measures such as treatment with ketamine, ECT, antidepressants or with 5-HT2A receptor agonists (such as psilocybin).

In the context of the present invention, the term "AIIt complex" shall relate to a complex comprising of proteins that is expressed on the cell surface of a variety of cells and functions as a plasminogen receptor. In mammals. AIIt consists of two molecules of Annexin A2 bound together by a dimer of the protein S100A10. Annexin A2 contains phospholipid-binding sites that anchor S100A10 to the cell surface membrane, whereas the C-terminal lysine residue of S100A10 binds tPA and plasminogen. S100A10 has also been shown to colocalize plasminogen with the uPA/uPAR complex. Therefore, the AIIt complex according to the present invention comprises the proteins Annexin A2 (2 subunits), S100A10 (dimer), tPA, plasminogen, plasmin. uPA, and uPAR, preferably the proteins ANXA2 (2 subunits), S100A10 (dimer), tPA, plasminogen, and plasmin. S100A10-induced colocalization of plasminogen with its activators accelerates the proteolytic cleavage of plasminogen, resulting in plasmin generation and therefore enhanced fibrinolytic activity.

These therapeutic measures are aimed at triggering biological signal cascades which, among other things, individually influence the receptor density, the affinity of the substances for the receptors or the release of neurotransmitters depending on the genetic disposition of the patient. This is possible and stable over the long term by monitoring the plasminogen level and/or the tPA and/or uPA concentration and the plasmin level. In addition, plasminogen and/or tPA and/or uPA and/or plasmin can have a direct therapeutic effect, i.e., without additional combination therapy with, for example, 5-HT2A receptor agonists, ketamine, SSRI antidepressant, mGluR2 agonist, mGluR2/3 agonist, positive allosteric modulator (PAM), cytokine, and/or ECT.

In the context of the present invention, the term "PAM" shall relate to compounds as allosteric modulators that have little or no detectable functional activity when bound to the target protein in the absence of an orthosteric agonist (i.e. a compound or protein or functional fragment thereof as described herein), but can potentiate the activity of bound orthosteric agonist, seen as an increase in apparent potency and/or efficacy of the orthosteric agonist (see, for example⁴⁵, Burford NT, Traynor JR, Alt A. Positive allosteric modulators of the µ-opioid receptor: a novel approach for future pain medications. Br J Pharmacol. 2015 Jan;172(2):277-86. doi: 10.1111/bph.12599. Epub 2014 Jul 1. PMID: 24460691; PMCID: PMC4292945). Assays to determine PAMs are known to the skilled person and described in the state of the art.

In the context of the present invention, the term "tPA" shall relate to the protein tissue plasminogen activator (abbreviated tPA or PLAT) that is involved in the breakdown of blood clots. As an enzyme, it catalyzes the conversion of plasminogen to plasmin, the major enzyme responsible for clot breakdown. The amino acid sequence of human tPA can be found at NP_000921 (562 aa, status 23-JAN-2023) in the form of tissue-type plasminogen activator isoform 1 preproprotein. A preferred amino acid sequence of the tPA protein for use according to the present invention comprises an amino acid sequence that is to at least 90%, preferably to at least 95%, and most preferred to at least 99% or even to 100% identical to the amino acids of the human tPA protein. Orthologs of the tPA amino acid sequence according to the present invention may be selected from bovine, dog, equine, cat, chicken, monkey, mouse, and rat. tPA can be manufactured using recombinant biotechnology techniques; tPA produced by such means are referred to as recombinant tissue plasminogen activator (rtPA). Specific rtPAs include alteplase (527 aa), reteplase (355 aa of the native tPA), and Tenecteplase (glycoprotein of 527 amino acids, sequence is modified at three positions compared to native human tPA).

tPA is registered by the FDA for a treatment of myocardial infarction (acute myocardial infarct⁴⁶⁻⁴⁸ as well as stroke (acute ischemic stroke,^{49,50}, see also: https://www.ninds.nih.gov/about-ninds/impact/ninds-contributions-approved-therapies/tissue-plasminogen-activator-acute-ischemic-stroke-alteplase-activaser, date 17.03.2023). Recombinant tPA can already be produced in commercially relevant amounts⁵¹. It was shown in mouse models of stroke that a nasal application of tPA leads to a functional improvements and neuronal remodeling^{52,53}. Since has a short half-life of only 5-10 minutes in the blood, nasal application is a good possibility to increase the therapeutic window. Nevertheless, there is evidence that tPA could also be neurotoxic and increases the risk of bleedings, respectively⁵⁴.

In the context of the present invention, the term "uPA" shall relate to the protein urokinase-type plasminogen activator (abbreviated uPA or PLAU). As an enzyme, it catalyzes the conversion of plasminogen to plasmin, the major enzyme responsible for clot breakdown. Urokinase is a 411-residue protein, consisting of three domains: the serine protease domain (consisting of residues 159-411), the kringle domain (consisting of residues 50-131), and the EGF-like domain (consisting of residues 1-49). The amino acid sequence of human uPA can be found at NP_002649.2 (431 aa, status 22-JAN-2023) in the form of urokinase-type plasminogen activator isoform 1 preproprotein. A preferred amino acid sequence of the uPA protein for use according to the present invention comprises an amino acid sequence that is to at least 90%, preferably to at least 95%, and most preferred to at least 99% or even to 100% identical to the amino acids of the human uPA protein. Orthologs of the uPA amino acid sequence according to the present invention may be selected from bovine, dog, equine, cat, chicken, monkey, mouse, and rat. uPA can be manufactured using recombinant biotechnology techniques.

In the context of the present invention, the term "uPAR" shall relate to the protein urokinase-type plasminogen activator receptor (abbreviated uPAR or PLAUR or CD87). uPAR is a multidomain glycoprotein tethered to the cell membrane with a glycosylphosphotidylinositol (GPI) anchor. uPAR was originally identified as a saturable binding site for urokinase (uPA) on the cell surface. When urokinase is bound to the receptor, there is cleavage between the GPI-anchor and the uPAR, releasing a soluble form of the protein known as suPAR. The amino acid sequence of human PLAUR can be found at NP_002650.1 (335 aa, status 22-JAN-2023) in the form of urokinase plasminogen activator surface receptor isoform 1 preproprotein. A preferred amino acid sequence of the uPAR protein as used according to the present invention comprises an amino acid sequence that is to at least 90%, preferably to at least 95%, and most preferred to at least 99% or even to 100% identical to the amino acids of the human uPAR protein. Orthologs of the uPAR amino acid sequence according to the present invention may be selected from bovine, dog, equine, cat, chicken, monkey, mouse, and rat. uPAR can be manufactured using recombinant biotechnology techniques, it has been possible to express uPAR recombinantly in CHO-cells and S2 cells from *Drosophila melanogaster.*

In the context of the present invention, the term "plasminogen receptor" shall relate to the protein plasminogen receptor (abbreviated PLGRKT). PLGRKT is a cell-surface plasminogen (PLG; 173350) receptor that enhances plasminogen activation and regulates catecholamine release. It also plays a major functional role in plasminogen-dependent monocyte/macrophage migration, invasion, and recruitment in the inflammatory response. Plg-R_{KT} is expressed by immune cells, including monocytes and macrophages. The amino acid sequence of human PLGRKT can be found at NP_060935 (147 aa, status 18-DEC-2022). A preferred amino acid sequence of the PLGRKT protein as used according to the present invention comprises an amino acid sequence that is to at least 90%, preferably to at least 95%, and most preferred to at least 99% or even to 100% identical to the amino acids of the human PLGRKT protein. Orthologs of the PLGRKT amino acid sequence according to the present invention may be selected from bovine, dog, equine, cat, monkey, mouse, and rat.

In the context of the present invention, the term "plasmin" shall relate to the protein plasmin involved in fibrinolysis. In humans, the plasmin protein (in the zymogen/proenzyme form of plasminogen) is encoded by the *PLG* gene. The amino acid sequence of human plasminogen can be found at B2R7F8 (UniProt database, 810 aa). In the context of the present invention, the term "plasminogen" shall relate to the proenzyme plasminogen that is released (PLG) from the liver into the systemic circulation. Two major glycoforms of plasminogen are present in humans - type I plasminogen contains two glycosylation moieties (N-linked to N289 and O-linked to T346), whereas type II plasminogen contains only a single O-linked sugar (O-linked to T346). Type II plasminogen is preferentially recruited to the cell surface over the type I glycoform. Conversely, type I plasminogen appears more readily recruited to blood clots. The conversion of plasminogen to plasmin involves the cleavage of the peptide bond between Arg-561 and Val-562. A preferred amino acid sequence of the plasminogen or plasmin protein for use according to the present invention comprises an amino acid sequence that is to at least 90%, preferably to at least 95%, and most preferred to at least 99% or even to 100% identical to the amino acids of the human plasminogen or plasmin protein. Orthologs of the plasminogen amino acid sequence according to the present invention may be selected from bovine, dog, equine, cat, chicken, monkey, mouse, and rat.

Plasminogen is already used in the therapy of stroke⁵⁵ - In addition there are reports about a use of plasminogen in certain neurodegenerative diseases (use at spinal muscular atrophy and Morbus Parkinson) - In 2021 plasminogen was registered by the FDA for the treatment of patients with hypoplasminogenemia (https://www.fda.gov/news-events/press-announcements/fda-approves-first-treatment-patients-plasminogen-deficiency-rare-genetic-disorder, date 17.03.2023). First animal experiments show that also plasmin can be safely used and in much higher dose than tPA without the risk of bleedings⁵⁶. Currently, clinical tests are performed for plasmin in the treatment of stroke⁵⁷.

The above-mentioned results show that the bioavailability of plasminogen, tPA, uPA, and plasmin can be manipulated using external application. Both plasminogen and tPA are registered for therapeutic applications in human treatment, plasmin is currently tested.

In the context of the present invention, the term "functional fragment of the uPA, tPA, plasminogen or plasmin protein", respectively shall relate to a part of the amino acid sequences of the above proteins that is truncated at the N- and/or C-terminus of the amino acid sequence, but still performs and/or supports (e.g. as a PAM) its function as required/desired in the context of the present invention, namely - when expressed, translated and/or administered - causes prevention or treatment of a psychiatric disorder or disease as disclosed herein and/or improves or amplifies the effect of 5-HT2A receptor agonists, ketamine, SSRI antidepressant, mGluR2 agonist, mGluR2/3 agonist, positive allosteric modulator (PAM), cytokine, and/or ECT, and other psychoplastogenic substances in a subject.

In the context of the present invention, the term "mRNA" shall relate to a respective nucleic acid molecule comprising a nucleic acid sequence encoding for the uPA, tPA, plasminogen or plasmin protein or functional fragment thereof as above. The mRNA molecule can be natural or produced synthetically or in vitro, or combinations thereof. The mRNA may comprise modified bases and/or DNA or other types of nucleic acid components, as long as the desired function(s) (see above) is/are not substantially impaired. In the context of the invention, in general and also preferably an mRNA as used comprises the same structural components as natural mRNA in eukaryotic cells. It has at least a 5' cap, a 5'-untranslated region (UTR), a 3'-UTR, an open reading frame_(ORF), which encodes the relevant Flt3L protein or functional fragment thereof, for example as adjuvant or antigen, and a 3'-poly(A) tail. Preferably, the term further includes mRNAs comprising nucleotide sequences that are identical or at least 80%, preferably at least 90%, more preferably at least 95% identical to the nucleotide sequence of an mRNA encoding at least one of the mammalian uPA, tPA, plasminogen or plasmin protein, in particular a human protein. The nucleic acid molecule may be polycistronic.

In the context of the present invention, it was surprisingly found that a synergistic effect on the neuroplasticity of human neuronal cells can be achieved when applying 5-HT2A receptor agonists together with a modulation of the fibrinolytic cascade. Thus, plasminogen/plasmin/tPA/uPA are linked with significant effects on human neuronal cells, and this puts the AIIt complex and/or PLGRKT, which is directly involved in the process, into the spotlight of further developments.

There are some publications that remotely link the main component of the complexes, p 11, and the key enzyme tPA with neuroplasticity^{13,34,58-69}. Reduced levels were associated with the development of depression^{5,13,34,67}. Several publications indicate that reduced levels of tPA, BDNF and p11 can be elevated in depressive patients using electroconvulsive therapy (ECT) or treatment with psychopharmaceutic compounds, such as, for example antidepressive agents (e.g., Selective Serotonin Reuptake Inhibitors (SSRI))^{5,8,13,15,31-44,67}.

Psychedelic compounds and serotonergic 5-HT2A receptor agonists, such as psilocybin and LSD, but also the N-methyl-d-aspartate (NMDA) receptor agonist ketamine, are already used in clinical studies for their effectivity in the treatment of psychiatric diseases, such as depression and addiction, and so far, show promising results⁷⁰⁻⁷³. Nevertheless, the exact mechanism of how 5-HT2A receptor agonists mediate their therapeutic effect is not understood. It could be shown that, amongst others, these so-called *"psychoplastogens*"⁷⁴ can improve the neuronal plasticity through also upregulating the cellular levels of BDNF, tPA and p11^{14,74-90}. This renders therapeutic applications that modulate an increase of cellular m-BDNF as well as p11 and tPA-levels highly interesting.

In general, any form of the at least one protein for use according to the present invention can be used, as long as it achieves or substantially achieves the desired therapeutic or preventive effect. Preferred is the at least one protein or functional fragment thereof for use according to the present invention, wherein the protein or functional fragment thereof has been recombinantly produced. Preferred examples for tPA are mentioned above, and include alteplase (527 aa), reteplase (355 aa of the native tPA), and Tenecteplase (glycoprotein of 527 amino acids, sequence is modified at three positions compared to native human tPA). Recombinant plasminogen is also available, for example from *recombinant* human *plasminogen protein* overexpression cell lysate, derived from transfected HEK293 cells. Therefore, the person of skill is readily able to produce the recombinant proteins or functional fragments thereof, also in their glycosylated forms.

In another embodiment of the present invention, the at least one nucleic acid or functional fragment thereof for use according to the present invention is RNA, DNA, or mixtures thereof, is in the form of naked RNA, an expression cassette or vector, or has been recombinantly or synthetically produced or in vitro transcribed. In general, any suitable nucleic acid can be used to provide the nucleic acid or functional fragment thereof for use according to the present invention to a subject or a cell. Administration as a naked mRNA or an RNA in the form of a coated nucleic acid or as a particle comprising the nucleic acid can be achieved (for reviews, see⁹¹ and⁹² Kulkarni JA, Witzigmann D, Thomson SB, Chen S, Leavitt BR, Cullis PR, van der Meel R. The current landscape of nucleic acid therapeutics. Nat Nanotechnol. 2021 Jun; 16(6):630-643. doi: 10.1038/s41565-021-00898-0. Epub 2021 May 31. Erratum in: Nat Nanotechnol. 2021 Jul;16(7):841. PMID: 34059811). The person of skill is readily able to produce the at least one nucleic acid or functional fragment thereof for use according to the present invention.

Preferred is the at least one protein or functional fragment thereof or the at least one nucleic acid or functional fragment thereof for use according to the present invention, wherein the subject suffers from a psychiatric disorder or disease that is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

As disclosed herein, the available amounts of the substrate plasminogen or uPA, tPA as well as plasmin itself have a direct effect on the ratio of pro-BDNF to m-BDNF. Therefore, the identification of compounds that modulate and in particular increase the expression and/or protein level (amount) as well as the biological function of components of the AIIt complex (including in particular PAUR), and/or PLGRKT would be useful i) to "directly" prevent or treat psychiatric conditions or diseases as herein, and/or ii) can be useful as combination prevention or therapy in order to increase or amplify the effect of therapeutic measures, such as the treatment with ketamine, ECT, antidepressive agents or 5-HT2A receptor agonists (such as psilocybin), SSRI antidepressants, mGluR2 agonists, mGluR2/3 agonists, positive allosteric modulators (PAMs), cytokines, and/or ECT. Therefore, another important aspect of the present invention is a method for identifying a compound that increases the expression, amount and/or biological activity of a protein of the AIIt complex and/or PLGRKT in a cell of a subject. Preferably, the cell is a mammalian cell, such as a neuronal cell, such as, for example, a human induced pluripotent stem cell-derived neuronal cell.

The method according to the present invention comprises the first step of i) contacting at least one candidate compound with a cell that expresses at least one protein of the AIIt complex and/or PLGRKT. Contacting can be done, for example, in culture, on a plate or on a solid surface, depending on the cells as used and the desired analysis method(s) to follow. The contacting may be in vivo or in vitro, in solution or comprises the candidate compound molecule bound or conjugated to a solid carrier. Respective formats are also described in the art, and known to the person of skill. The second step comprises detecting an increase of the expression, amount and/or biological activity of the at least one protein of the AIIt complex and/or PLGRKT in the cell in the presence of the candidate compound compared to the expression, amount and/or biological activity in the absence of the candidate compound. Methods to detect expression are well-known to the person of skill and may comprise quantitative rtPCR analysis or an analysis using antibodies or stainings, mass spectrometry, NMR assays, pull-down assays, and the like. Similarly, the amount may be detected, for example using antibodies or stainings. The individual biological activities of the components differ from the individual component. Tests regarding an increase of tPA, uPA, or plasminogen may involve detecting the fibrinolytic activity, nevertheless, preferred is a detection of a change in the ratio of pro-BDNF to m-BDNF in the assay/cell in the absence or presence of the at least one compound. Components of the assay(s), such as the compound and/or the at least one protein of the AIIt complex and/or PLGRKT may be suitably labelled for the assays, preferably in the cell. In a particularly preferred embodiment, proteins like Annexin A2 and S100A10 can be tagged endogenously using fluorescent dyes and a CRISPR/CAS-based genetic modification system (see, for example⁹³. Preferred is a method according to the present invention, wherein the at least one protein of the AIIt complex is selected from the group consisting of S100A10, ANXA2 (Annexin 2), tPA, plasminogen, and plasmin. In the final step of the method according to the present invention, a modulation, preferably an increase of the expression, amount and/or biological activity of the at least one protein of the AIIt complex and/or PLGRKT in the cell identifies a compound that increases the expression, amount and/or biological activity of a protein of the AIIt complex and/or PLGRKT in a cell of a subject.

In the context of the present invention, the candidate compound can be selected from the group consisting of a chemical organic molecule, a molecule selected from a library of small organic molecules (molecular weight less than about 500 Da), a molecule selected from a combinatory library, a cell extract, in particular a plant cell extract, a small molecular drug, a protein, a protein fragment, a molecule selected from a peptide library, an antibody or antigen binding fragment thereof, or a nucleic acid, a DNA, an RNA, or an siRNA.

These candidate molecules may also be used as a basis to screen for improved compounds. Thus, preferred is the method according to the present invention, wherein after the identification of the pro-BDNF to m-BDNF modifying compound and/or a change of the fibrinolytic activity, the method further comprises the step of chemically modifying the compound. In general, many methods of how to modify compounds of the present invention are known to the person of skill and are disclosed in the literature.

Modifications of the compounds will usually fall into several categories, for example a) mutations/changes of amino acids into different amino acids, b) chemical modifications, e.g., through the addition of additional chemical groups, c) changes of the size/length of the compound, and/or d) the attachment of additional groups to the molecule (including marker groups, labels, linkers or carriers, such as chelators). In a next step, the modified compound is tested again in at least one of tests as above, and if the property of the compound is improved compared to its unaltered state. In the context of the present invention, an "improved" activity relates to an increase of m-BDNF and/or fibrinolytic activity.

Preferred is the method according to the present invention, wherein the contacting is performed in the presence of at least one psychoplastogenic agent, 5-HT2A receptor agonist, ketamine, SSRI antidepressant, cytokine, mGluR2 agonist, mGluR2/3 agonist, positive allosteric modulator (PAM), and/or ECT. This combination assay identifies compounds that have an effect on the increase of m-BDNF and/or fibrinolytic activity in addition to the therapeutics and is also suitable to identify potentially occurring synergistic improvements. Of course, also the identifying is then done in the presence of the additional compound.

Preferred is the method according to the present invention, wherein the compound as identified specifically increases the expression, amount and/or biological activity of at least one protein of the AIIt complex (including in particular PAUR) and/or PLGRKT.

Another aspect of the present invention relates to a method for producing a pharmaceutical composition, comprising performing a method for identifying according to the present invention, and admixing the compound as identified with at least one pharmaceutically carrier (see also below).

Another aspect of the present invention relates to a pharmaceutical composition comprising at least one protein or functional fragment thereof, at least one nucleic acid or functional fragment thereof, at least one expression cassette or at least one vector for use according to the present invention, together with at least one pharmaceutically acceptable carrier, or as produced according to the present invention, for use in medicine. Preferred is a pharmaceutical composition for use according to the present invention for use in the prevention or treatment of a psychiatric disorder or disease in a subject.

Preferably, said composition comprises an aqueous formulation. The term "pharmaceutical composition" refers to a preparation which is in such form as to permit the biological activity of an active ingredient contained therein (here, the at least one protein) to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g., by injection or infusion). Preferred is the pharmaceutical composition for use according to the present invention, wherein the composition is for injection, oral and/or nasal application.

The pharmaceutical compositions for use according to the invention may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The pharmaceutical compositions according to the invention may be in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray. Preferred is an injectable composition.

In some embodiments, the composition comprises the active ingredient, such as the at least one protein or functional fragment thereof, at least one nucleic acid or functional fragment thereof, at least one expression cassette or at least one vector at a concentration in the range of about 1 mg/ml to about 2 mg/ml, 3 mg/ml, 4 mg/ml, 5 mg/ml, 6 mg/ml, 7 mg/ml, 8 mg/ml, 9 mg/ml, 10mg/ml, 11 mg/ml, 12 mg/ml, 13 mg/ml, 14 mg/ml, 15 mg/ml, 16 mg/ml, 17 mg/ml, 18 mg/ml, 19 mg/ml or 20 mg/ml. In some embodiment, the composition further comprises one or more additional therapeutic agents, e.g., second, third or fourth therapeutic agents, for example selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent.

The pharmaceutical composition for use according to the present invention is therefore preferably used in a combination therapy or prevention. Preferred examples are pre-treatment to "prime" the AIIt complex and/or PLGRKT by specifically increasing the expression, amount and/or biological activity of at least one protein of the AIIt complex and/or PLGRKT, to improve the effect of the therapeutic agents, for example selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent. The combination can be administered together or in separate doses, as long as the effects of the pharmaceuticals lead to a combinatory effect of the drugs as applied.

Preferred is the pharmaceutical composition for use according to the present invention, wherein the use is for an improvement or amplification of the therapeutic effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent in the prevention or treatment of a psychiatric disorder or disease in a subject. Further preferred is the pharmaceutical composition for use according to the present invention, wherein the use is for an improvement or amplification of the therapeutic effect of electroconvulsive therapy (ECT) and/or psychotherapy in a subject.

Even further preferred is the pharmaceutical composition for use according to the present invention, wherein the use is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.

Preferred is the pharmaceutical composition for use according to the present invention, wherein the subject suffers from a psychiatric disorder or disease that is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

In the context of the present invention, the term "synergistic effect" shall mean the result of two or more compounds or processes as disclosed herein interacting together to produce an effect that is greater than the cumulative effect that those processes produce when used individually.

Another aspect of the present invention relates to the use of at least one of a protein selected from group consisting of uPA, tPA, plasminogen, plasmin, and functional fragments thereof, or a nucleic acid encoding said uPA, tPA, plasminogen, plasmin, and functional fragments thereof for improving or amplifying the therapeutic effect of electroconvulsive therapy (ECT), psychotherapy or for improving or amplifying the therapeutic effect of a compound in the prevention or treatment of a psychiatric disorder or disease in a subject.

Preferred is the use according to the present invention, wherein the protein uPA, tPA and plasminogen or functional fragments thereof, or the nucleic acid encoding said uPA, tPA and plasminogen or functional fragments thereof are used in combination as disclosed herein, and in particular above.

Another aspect of the present invention relates to a method for improving or amplifying the therapeutic effect of electroconvulsive therapy (ECT), psychotherapy or a compound in the prevention or treatment of a psychiatric disorder or disease in a subject comprising administering an effective amount of at least one of a protein selected from group consisting of uPA, tPA, plasminogen, plasmin, and functional fragments thereof, or a nucleic acid encoding said uPA, tPA, plasminogen, plasmin, and functional fragments thereof.

Preferred is the method according to the present invention, wherein the protein uPA, tPA and plasminogen or functional fragments thereof, or the nucleic acid encoding said uPA, tPA and plasminogen or functional fragments thereof are administered in combination as disclosed herein, and in particular above.

In the context of the present invention, the term "about" shall mean to include a deviation of +/- 10% of the value as given, if not indicated otherwise.

In the context of the present invention, a "subject" relates to a mammal, animal or individual, such as a person or patient that undergoes prevention or treatment according to the invention against a psychiatric disorder or disease.

Yet another aspect of the present invention relates to a method for preventing or treating a psychiatric disorder or disease in a subject in need of said prevention or treatment, comprising administering to the subject an effective amount of at least one of a protein selected from group consisting of uPA, tPA, plasminogen, plasmin, and functional fragments thereof, or a nucleic acid encoding said uPA, tPA, plasminogen, plasmin, and functional fragments thereof. Details regarding the method of treatment are similar to the medical uses as above.

Preferred is the method according to the present invention, wherein the protein uPA, tPA and plasminogen or functional fragments thereof, or the nucleic acid encoding said uPA, tPA and plasminogen or functional fragments thereof are administered in combination as disclosed herein, and in particular above.

In the context of the present invention, the term "treatment" or "therapy" shall mean the attempted remediation of a health problem as disclosed herein, i.e., the psychiatric disorder or disease in the subject.

Preferred is the method according to the present invention, wherein the protein or functional fragment thereof has been recombinantly produced.

Further preferred is the method according to the present invention, wherein the nucleic acid or functional fragment thereof is RNA, DNA, or mixtures thereof, is in the form of naked RNA, an expression cassette or vector, or has been recombinantly or synthetically produced or in vitro transcribed.

Further preferred is the method according to the present invention, wherein the at least one protein or functional fragment thereof, at least one nucleic acid or functional fragment thereof, the at least one expression cassette or at least one vector are administered as a pharmaceutical composition together with at least one pharmaceutically acceptable carrier, or as a pharmaceutical composition produced according to the present invention. The composition can be administered by injection, oral and/or nasal application. Details regarding the pharmaceutical composition and the uses thereof are also described above.

Further preferred is the method according to the present invention, wherein the prevention or treatment comprises an improvement or amplification of the effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent or an improvement or amplification of the therapeutic effect of electroconvulsive therapy (ECT) and/or psychotherapy in the subject.

Further preferred is the method according to the present invention, wherein the treatment is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.

Even further preferred is the method according to the present invention, wherein the psychiatric disorder or disease in the subject is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

In studies of induced pluripotent stem cells (iPSCs), the inventors have found that stimulation of human neurons with the 5-HT2A receptor agonist psilocin (as a psychoactive component of psilocybin) leads to upregulation of the core components of the AIIt complex (annexin A2 and p 11) and the plasminogen-processing enzymes tPA and uPA (urokinase-type plasminogen activator) (Figure 1). Furthermore, the genes of the receptors of tPA (PLGRKT) and uPA (PLAUR) are upregulated (Figure 3C). This is of particular interest because the plasmin rate is significantly positively correlated with the number of plasminogen receptors⁴. Based on the hypothesis that this leads to an increased cleavage of plasminogen into plasmin, which in turn changes the ratio of pro-BDNF to m-BDNF, the inventors conducted various investigations as disclosed herein.

The inventors were able to show that when plasminogen is added to the cell culture supernatant and treated simultaneously with the 5-HT2A receptor agonist psilocin, m-BDNF is increasingly processed from artificial pro-BDNF (Figure 3). As a result, the proportion of artificial pro-BDNF decreases. Treatment with plasminogen alone also leads to an increase in endogenous pro-BDNF and endogenous m-BDNF, especially after psilocin treatment. At the same time, the inventors were able to show that the total proportion of endogenous BDNF, as well as endogenous pro-BDNF, increases in the cells after psilocin treatment. The 5-HT2A receptor agonist psilocin thus led to an increased efficacy of the AIIt complex, which leads to an increased turnover of plasminogen in plasmin and thus from pro-BDNF to m-BDNF with simultaneous upregulation of tPA and uPA and the substrate pro-BDNF. This increases the endogenous overall level of BDNF but above all the m-BDNF level associated with synaptic plasticity. Since these enzyme cascades work depending on the availability of the corresponding substrates and enzymes, an increased availability of components of the AIIT complex, uPA, plasminogen and/or tPA and/or plasmin directly affects the ratio of pro-BDNF and m-BDNF.

On the basis of these observations, the inventors postulate the following therapeutic approaches.
1. Treatment of psychiatric disorders and diseases by activation of the AIIt complex and/or PLGRKT by i) substances that affect the expression and / or protein level of components of the AIIt complex and/or PLGRKT, by ii) oral, intravenous or intranasal administration of uPA and/or tPA and/or iii) by oral, intravenous or intranasal administration of plasminogen or iv) by oral, intravenous or intranasal administration of plasmin. All four strategies can be used individually or in combination.
2. Adjuvant therapy using 5-HT2A receptor agonists and/or other substances affecting the AIIt complex and/or PLGRKT or therapeutic measures such as ketamine, antidepressants or ECT together with plasminogen and/or uPA and/or tPA and/or plasmin to improve or enhance the efficacy of 5-HT2A receptor agonists or similar substances affecting the AIIt complex and/or PLGRKT. Can also be performed as a combination therapy for example with at least one 5-HT2A receptor agonist, ketamine, SSRI antidepressant, mGluR2 agonist, mGluR2/3 agonist, positive allosteric modulator (PAM), cytokine, and/or ECT, and/or together with psychotherapy. Preferably the improvement or amplification of the therapeutic effect is synergistic.

Manipulation of BDNF as a therapeutic concept has been discussed for some years in the treatment of psychiatric disorders³⁰. Several forms of therapy, including antidepressants, ECT and ketamine treatment, have been shown to upregulate BDNF and tPA^{13,32-35}. Together, this can change the ratio of pro-BDNF to m-BDNF via the plasminogen-plasmin cascade. No one has yet postulated plasminogen and/or uPA and/or tPA and/or plasmin as a therapeutic agent for the treatment of psychiatric disorders. In addition, the present results show that 5-HT2A receptor agonists, which represent a novel therapeutic concept, activate not only BDNF and tPA, but all components of the AIIt complex and PLGRKT.

The available amount of the substrate plasminogen or tPA or uPA thus directly affects the availability of plasmin and thus the ratio of pro-BDNF to m-BDNF. An increase in the bioavailability of the nuclear enzyme plasmin also leads to an increased processing of pro-BDNF into m-BDNF. This is also a new combinatorial measure to enhance the effectiveness of therapeutic measures such as treatment with ketamine, ECT, antidepressants or with 5-HT2A receptor agonists (such as psilocybin).

In conclusion, the inventor's results show that the bioavailability of plasminogen, uPA, tPA and plasmin can be manipulated via external application. Both plasminogen and tPA are approved for therapeutic applications in human medicine. Plasmin is in clinical testing, and thus their effectiveness and tolerability in humans have already been tested for the approved therapeutic applications.

The present invention relates to the following items:
Item 1. At least one of a protein selected from group consisting of tPA, uPA, plasminogen, plasmin, and functional fragments thereof, or a nucleic acid encoding said tPA, uPA, plasminogen, plasmin, and functional fragments thereof for use in the prevention or treatment of a psychiatric disorder or disease in a subject.
Item 2. The at least one protein for use according to Item 1, wherein the protein tPA, uPA and plasminogen or functional fragments thereof, or the nucleic acid encoding said tPA, uPA and plasminogen or functional fragments thereof are used in combination.
Item 3. The at least one protein for use according to Item 1 or 2, wherein the protein or functional fragment thereof has been recombinantly produced.
Item 4. The at least one nucleic acid for use according to Item 1 or 2, wherein the nucleic acid or functional fragment thereof is RNA, DNA, or mixtures thereof, is in the form of naked RNA, an expression cassette or vector, or has been recombinantly or synthetically produced or in vitro transcribed.
Item 5. A method for identifying a compound that increases the expression, amount and/or biological activity of a protein of the AIIt complex and/or PLGRKT in a cell of a subject, comprising the steps of i) contacting at least one candidate compound with a cell that expresses at least one protein of the AIIt complex and/or PLGRKT, and ii) detecting an increase of the expression, amount and/or biological activity of the at least one protein of the AIIt complex and/or PLGRKT in the cell in the presence of the candidate compound compared to the expression, amount and/or biological activity in the absence of the candidate compound, optionally in the presence of at least one 5-HT2A receptor agonist, ketamine, SSRI antidepressant, cytokine, and/or ECT, wherein an increase of the expression, amount and/or biological activity of the at least one protein of the AIIt complex and/or PLGRKT in the cell identifies a compound that increases the expression, amount and/or biological activity of a protein of the AIIt complex and/or PLGRKT in a cell of a subject.
Item 6. The method according to Item 5, wherein the at least one protein of the AIIt complex and/or PLGRKT is selected from the group consisting of S100A10, ANXA2, tPA, uPA, PAUR, PLGRKT, plasminogen, and plasmin, and/or wherein the compound specifically increases the expression, amount and/or biological activity of one protein of the AIIt complex.
Item 7. The method according to Item 5 or 6, wherein the cell is a neuronal cell, such as, for example, a human induced pluripotent stem cell-derived neuronal cell.
Item 8. The method according to any one of Items 5 to 7, wherein the compound is selected from the group consisting of a natural compound, a plant extract, a peptide, a protein, a small molecule (less than about 500 Da), a nucleic acid, a DNA, an RNA, an siRNA, and an antibody or antigen binding fragment thereof.
Item 9. A method for producing a pharmaceutical composition, comprising performing a method according to any one of Items 5 to 8, and admixing the compound as identified with at least one pharmaceutically carrier.
Item 10. A pharmaceutical composition, comprising at least one protein or functional fragment thereof, at least one nucleic acid or functional fragment thereof, at least one expression cassette or at least one vector for use according to any one of Items 1 to 4, together with at least one pharmaceutically acceptable carrier, or as produced according to Item 9, for use in medicine, in particular for use in the prevention or treatment of a psychiatric disorder or disease in a subject.
Item 11. The pharmaceutical composition for use according to Item 10, wherein the composition is for injection, oral and/or nasal application.
Item 12. The pharmaceutical composition for use according to Item 10 or 11, wherein the use is for an improvement or amplification of the therapeutic effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent in the prevention or treatment of a psychiatric disorder or disease in a subject.
Item 13. The pharmaceutical composition for use according to Item 10 or 11, wherein the use is for an improvement or amplification of the therapeutic effect of electroconvulsive therapy (ECT) and/or psychotherapy in a subject.
Item 14. The pharmaceutical composition for use according to Item 12 or 13, wherein the use is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.
Item 15. The use according to any one of Items 1 to 4 or the use according to any one of Items 10 to 14, wherein the psychiatric disorder or disease in the subject is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.
Item 16. The use of at least one of a protein selected from group consisting of tPA, uPA, plasminogen, plasmin, and functional fragments thereof, or a nucleic acid encoding said tPA, uPA, plasminogen, plasmin, and functional fragments thereof for improving or amplifying the therapeutic effect of electroconvulsive therapy (ECT), psychotherapy or a compound in the prevention or treatment of a psychiatric disorder or disease in a subject.
Item 17. Use according to Item 16, wherein the protein tPA, uPA and plasminogen or functional fragments thereof, or the nucleic acid encoding said tPA, uPA and plasminogen or functional fragments thereof are used in combination.
Item 18. A method for improving or amplifying the therapeutic effect of electroconvulsive therapy (ECT), psychotherapy or a compound in the prevention or treatment of a psychiatric disorder or disease in a subject comprising administering an effective amount of at least one of a protein selected from group consisting of tPA, uPA, plasminogen, plasmin, and functional fragments thereof, or a nucleic acid encoding said tPA, uPA, plasminogen, plasmin, and functional fragments thereof to said subject.
Item 19. The method according to Item 18, wherein the protein tPA, uPA and plasminogen or functional fragments thereof, or the nucleic acid encoding said tPA, uPA and plasminogen or functional fragments thereof are administered in combination.
Item 20. A method for preventing or treating a psychiatric disorder or disease in a subject in need of said prevention or treatment, comprising administering to a subject an effective amount of at least one of a protein selected from group consisting of tPA, uPA, plasminogen, plasmin, and functional fragments thereof, or a nucleic acid encoding said tPA, uPA, plasminogen, plasmin, and functional fragments thereof.
Item 21. The method according to Item 20, wherein the protein tPA, uPA and plasminogen or functional fragments thereof, or the nucleic acid encoding said tPA, uPA and plasminogen or functional fragments thereof are administered in combination.
Item 22. The method according to Item 20 or 21, wherein the protein or functional fragment thereof has been recombinantly produced.
Item 23. The method according to any one of Items 20 to 22, wherein the nucleic acid or functional fragment thereof is RNA, DNA, or mixtures thereof, is in the form of naked RNA, an expression cassette or vector, or has been recombinantly or synthetically produced or in vitro transcribed.
Item 24. The method according to any one of Items 20 to 23, wherein the at least one protein or functional fragment thereof, at least one nucleic acid or functional fragment thereof, the at least one expression cassette or at least one vector are administered as a pharmaceutical composition together with at least one pharmaceutically acceptable carrier, or as a pharmaceutical composition produced according to Item 9.
Item 25. The method according to Item 24, wherein the composition is administered by injection, oral and/or nasal application.
Item 26. The method according to any one of Items 20 to 25, wherein the prevention or treatment comprises an improvement or amplification of the effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent or an improvement or amplification of the therapeutic effect of electroconvulsive therapy (ECT) and/or psychotherapy in the subject.
Item 27. The method according to according to Item 26, wherein the treatment is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.
Item 28. The method according to any one of Items 20 to 27, wherein the psychiatric disorder or disease in the subject is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

The invention will now be described further in the following examples with reference to the accompanying figures, nevertheless, without being limited thereto. For the purposes of the present invention, all references as cited are incorporated by reference in their entireties.
Figure 1 shows the annexin A2 - S100A10 complex and its role in the extracellular generation of plasmin. The AIIT-complex consists of two annexin A2 proteins as well as one p11 (also known as S100A10) dimer. The complex binds the tissue plasminogen activator (tPA) and plasminogen, that is cleaved by tPA into plasmin. Also, uPA is associated. In the brain, in the extracellular space plasmin cleaves pro-BDNF into m-BDNF.

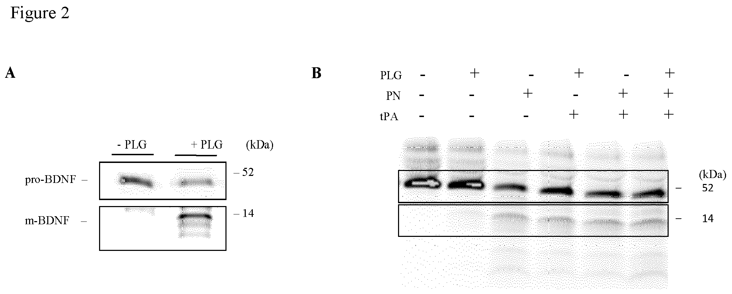

Figure 2 shows the influence of BDNF processing by plasminogen, tPA and/or plasmin in human brain cells *in vitro.* (A) an m-BDNF band becomes visible after the addition of plasminogen, showing that plasminogen is cleaved into plasmin by the cells. (B) Following the addition of plasminogen, plasmin as well as the combinations of plasminogen/tPA or plasmin/tPA or the triple combination of plasminogen/plasmin/tPA the processing of pro-BDNF into m-BDNF increases. PLG, plasminogen; PN, plasmin; tPA, tissue plasminogen activator.
Figure 3 shows that 5-HT2A receptor agonist function through the activation of the AIIt complex and processing of BDNF. (A) Upregulation of biological processes that are involved in the negative regulation of coagulation one day after psilocin administration, which (B) even after three days after psilocin administration stay upregulated 10-fold. In addition, biological processes, such as the "plasminogen activation", and "fibrinolysis" are upregulated 30-fold. (C) Genes that are involved in the composition of the annexin A2 - S100A10 complex as well as genes that are essential for the function of the complex function were significantly upregulated one day after psilocin gavage. The gene *ANXA2* encodes for annexin A2. S100A10 encodes for the p11 protein. Genes that encode for plasminogen-cleaving enzymes, such as PLAU (*urokinase plasminogen activator* (uPA) and *PLAT* (*tissue plasminogen activator* (tPA) were also significantly upregulated. In addition, the genes of the receptors of tPA (*PLGRKT*) and uPA (*PLAUR*) were significantly upregulated. (D-E) A representative Western Blot for a visualization of proteins showed an increase of P11 and annexin A2 protein levels three days after administration of psilocin (Psi), compared to control (Ctrl), which. (F-G) A representative immunofluorescence staining showed an increase of the annexin A2 - S100A10 co-localization three days after psilocin administration. Control (Ctrl) with *N* = 90 neurites, three days of psilocin administration (3d) with *N* = 90 neurites. Two control cell lines were included into the analysis. For all analyses Mann-Whitney-U test, respectively Kruskal-Wallis test (> 2 groups) for independent samples was calculated. Post hoc Wilcoxon rank sum test. Bonferroni-correction, adjusted ^{∗}*p* < .05, mean ± *SD.*

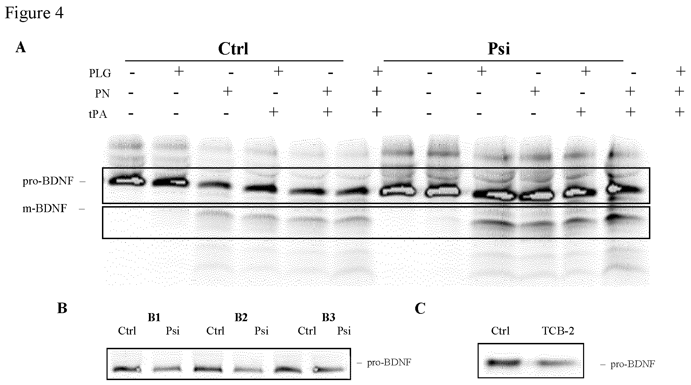

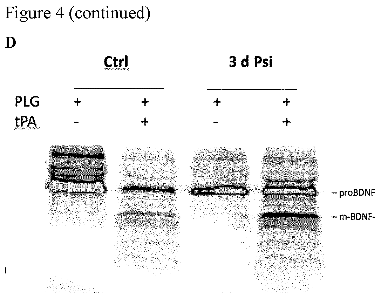

Figure 4 shows the potentiation of the effect of the BDNF processing of 5-HT2A receptor agonists by plasminogen, tPA and/or plasmin. (A) After addition of plasminogen, plasmin or the combinations of plasminogen/tPA or plasmin/tPA or a triple combination of plasminogen/plasmin/tPA, respectively, the processing of pro-BDNF in m-BDNF in the psilocin condition (Psi) is increased. (B-C) Reduction of the pro-BDNF level (B) in three independent experiments after psilocin pre-treatment for three days (Psi), and (C) reduction of the pro-BDNF level following pre-treatment with the selective 5-HT2A receptor agonist TCB-2. (D) Untreated and Psilocin treated neurons. tPA amplified the processing of BDNF, a pre-treatment with Psi amplifies this effect. PLG, plasminogen; PN, plasmin; tPA, tissue plasminogen activator.

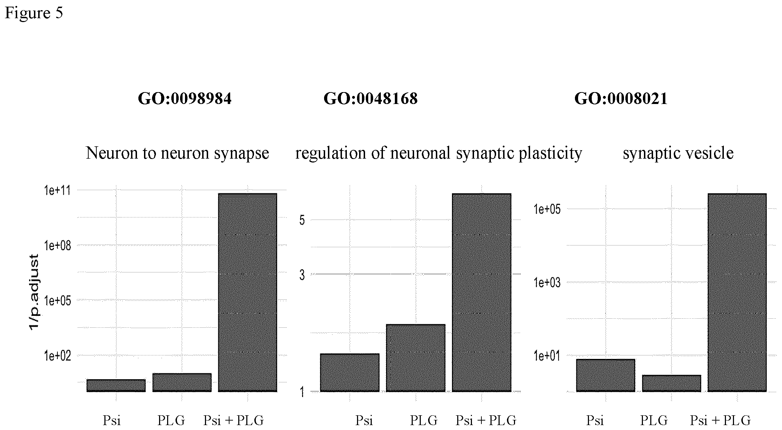

Figure 5 shows gene expression analyses of the effects of plasminogen and psilocin on synaptic plasticity. The monotreatment with psilocin (Psi) and plasminogen (PLG) shows significant enrichments of changes in gene expression in genetic groups (gene ontology, GO) that are associated with synaptic plasticity. The combination therapy of plasminogen and psilocin shows a synergistic effect on the changes in gene expression. The numbers as used for the GO groups are indicated.

### Examples

### Introduction/Summary

In the context of the present invention, the inventors provide experimental evidence that 5-HT2A receptor agonists (such as psilocin) activate the AIIt complex and furthermore PLGRKT. It is further shown that upon further addition of plasminogen and/or tPA or plasmin this activation leads to an increased turnover of pro-BDNF into m-BDNF in human brain cell cultures. The results were generated with human brain cells that were derived from pluripotent stem cells. The experiments show that a combination of compounds that (up)regulate the AIIt complex and/or also PLGRKT provide more than an additive effect and have a synergistic effect. This all was further confirmed by gene expression studies and immunoblotting.

### Materials and Methods

### Cell culture

The study forming the basis for the present examples was approved by the local ethics committee. All experiments with human material were in accordance with the Declaration of Helsinki. All healthy participants gave written informed consent.

Cells were cultured at 37°C, ambient O₂ and 5% CO₂ concentration under sterile conditions in an incubator (Binder). Applications with the cells were carried out in a sterile flow hood (Scanlaf Mars) by using sterile and autoclaved instruments and medium. Fibroblast-derived iPS cells were kept as colonies under feeder free-conditions in stem cell state on 5% (v/v) Geltrex^{™}-coated plates (Thermo Fisher Scientific) containing 1% (v/v) Pen/Strep (Thermo Fisher Scientific) in Essential 8 medium (DMEM/F12 with L-glutamine and HEPES (Thermo Fisher Scientific) supplemented with 1% (v/v) Pen/Strep (v/v), 64 µg/ml LAAP (Sigma-Aldrich), 14 ng/ml sodium selenite (Sigma-Aldrich), 200 ng/ml FGF-2 (154) (Cell Guidance Systems), 2 ng/ml TGF-β1 (Cell Guidance Systems), 20 µg/ml insulin (Sigma-Aldrich), 11 µg/ml transferrin (Sigma-Aldrich) that was changed daily. For passaging confluent iPS colonies were washed twice with phosphate buffered saline (DPBS) and then incubated with 0.5 mM EDTA (Thermo Fisher Scientific) for 5-10 min at RT until the colonies started to detach. After aspirating the EDTA solution the fractured colonies were gently resuspended in E8 medium containing 5 µM Rho-associated protein kinase (ROCK) inhibitor (Cell Guidance Systems).

For *in vitro* differentiation into neural progenitors E8 medium was replaced by neural progenitor induction medium phase 1 (advanced DMEM/F-12 medium with glutamine (Thermo Fisher Scientific), with 1% (v/v) Pen/Strep (v/v), 2 mM GlutaMAX^{™} (Thermo Fisher Scientific), 1% (v/v) B-27 supplement with RA (Thermo Fisher Scientific), 10 µM SB-431542 (Cell Guidance Systems), 1 µM LDN-193189 (Stemcell Technologies), 2 µM XAV939 (Cell Guidance Systems) and 5 µM cyclopamine (Cayman Chemical) when iPS cells reached 70-80% confluency. At day 4, cells were dissociated 1:2 to a monolayer, continue using aforementioned medium. Upon day 8 cells were dissociated 1:2 and medium was changed to induction medium phase 2 (advanced DMEM/F-12 medium with glutamine with 1% (v/v) Pen/Strep, 2 mM GlutaMAX^{™}, 1% (v/v) B-27 supplement with RA, 200 nM LDN-193189) for 8 more days. Finally, medium was changed to induction medium phase 3 (advanced DMEM/F-12 medium with glutamine with 1% (v/v) Pen/Strep, 2 mM GlutaMAX^{™}, 1% (v/v) B-27 supplement with RA, 20 ng/ml FGF-2 (147) (Cell Guidance Systems). Dissociation into single cells was done using TrypLE^{™} (Thermo Fisher Scientific) for 5 - 15 min at 37 °C until the cells start to detach. Cells were then transferred with wash medium to a 15 ml tube and centrifuged for 4 min at 1000 g. The pellet was gently resuspended in corresponding neural progenitor induction medium supplemented with 5 µM ROCK inhibitor.

*In vitro* differentiation into human cortical neurons was launched by the change to neural differentiation medium phase 1, defined as day 0 of differentiation. Neural differentiation phase 1 medium consisted of neural base medium 1 (DMEM/F-12 medium with glutamine, 0.1% (v/v) GlutaMAX^{™}, 1.8 mM CaCl₂ (Sigma-Aldrich), 1% (v/v) Pen/Strep, 1% (v/v) B-27, 0.5% (v/v) N2 supplement (prepared in the laboratory as follows: DMEM/F-12 with glutamine, 500 µg/ml insulin, 10 mg/ml transferrin, 520 ng/ml sodium selenite, 1.611 mg/ml putrescine (Sigma-Aldrich) and 630 ng/ml progesterone (Sigma-Aldrich)), 1% (v/v) NEAA (Thermo Fisher Scientific), 1.6 mg/ml glucose (Carl Roth)) supplemented with 200 µM ascorbic acid (Sigma-Aldrich), 1 µM LM22A (Sigma-Aldrich), 1 µM LM22B (Tocris Bioscience), 2 µM PD-0332991 (Selleckchem), 5 µM DAPT (Cell Guidance Systems) and was applied until day 3 of differentiation. The cells were split using TrypLE^{™} into phase 2 medium on polyethylenimine (Sigma Aldrich)/laminin (Sigma Aldrich) coated plates (1:2000 of 1% (PEI) in 25 mM boric acid (pH 8.4) (Thermo Fisher Scientific); 3.75 µg/ml laminin (Sigma Aldrich) in DPBS (Thermo Fisher Scientific). Neural differentiation medium phase 2-4 media consisted of neural base medium 2 with Neurobasal^{™} (Thermo Fisher Scientific) (plus 1.6 mg/ml glucose, 1% (v/v) Pen/Strep, 0.1% (v/v) GlutaMAX^{™} and 1% (v/v) B-27) and was supplemented additionally to supplements of phase 1 medium with 3 µM CHIR99021 (Cell Guidance Systems), 10 µM forskolin (Cell Guidance Systems) and 300 µM GABA (Sigma-Aldrich). At day 10 of differentiation medium was changed to medium phase 3 containing 3 µM CHIR99021. From day 17 on, CHIR99021 was removed in phase 4. At day 24 medium was changed to neural differentiation phase 5 medium in neural base medium 3 (advanced MEM (Thermo Fisher Scientific), 1.6 mg/ml glucose, 1% (v/v) Pen/Strep, 0.1% (v/v) GlutaMAX, 1% (v/v) B-27) and 0.27 nM Bryostatin 1 (Merck Millipore) for the next 14 days. Medium was changed every 3-4 days half. Treatment of cortical neurons started at day 42 of differentiation. 3 days prior to experiments, for drug dissolving and during course of the experiment culture medium was replaced with neural base medium 3 containing 1% (v/v) Pen/Strep, 1.6 mg/ml glucose, 0,1% (v/v) GlutMAX^{™}, 0,1% (v/v) B-27 and 200 µM ascorbic acid to avoid distorting effects of small molecules on cellular signal transduction pathways. Cells were treated permanently or for 10 min with 10 µM psilocin (THC-Pharm) or TCB-2 (Tocris Bioscience), washed once with advanced MEM containing 1% (v/v) Pen/Strep and were further cultured and then fixed or harvested after indicated time points post treatment.

### Western Blot: cleavage of recombinant pro-BDNF-Alexa-680 into m-BDNF-Alexa-680

100 µg of recombinant pro-BDNF (Alomone Labs) was labelled with the Alexa Fluor^{™} 680 Labeling Kit (Thermo Fisher Scientific) according to the manufacturer's instructions. Zeba^{™} Dye and Biotin Removal Spin Columns (Thermo Fisher Scientific) were used. Cortical neurons of a control condition and cells, that were previously treated for 72 hrs permanently with 10 µM psilocin or TCB-2, were incubated with recombinant 2.5 - 10 ng/µl plasminogen (R&D Systems), respectively with 1 ng/µl tPA (Sigma-Aldrich), plasmin (5U/mg) (Merck Millipore) for 1h at 37°C in an incubator. The supernatant was taken and treated with recombinant 20 - 80 ng/µl pro-BDNF-Alexa-680 for 5 - 6 hrs in a RT-PCR cycler. The supernatant was mixed with 6x denaturing protein sample buffer (93.75 mM Tris-HCL (pH 6.8), 6% SDS, 6% Glycerol, 9% 2-Mercaptoethanol, 0.25% bromophenol blue). 16.5% Mini-PROTEAN^{®} Tris-Tricine Gel (Bio-Rad Laboratories) were used for separation of proteins. The SDS-PAGE runs for about 30 min with 30 V for stacking of proteins and then for about 30 min at 110 V. The gel was directly visualized for Alexa-Fluor 680 with the Odyssey IR WB imaging system (Li-COR).

### RNA Bulk Sequencing

For Ribonucleic acid (RNA) isolation cells were (2 wells of 6well plate) resuspended in 500 µl peqGold Trifast^{™} (VWR) and incubated for 10 min at room temperature. 100 µl of chloroform (Sigma-Aldrich) was added to the lysate and incubated for 10 min at room temperature. Tubes were then centrifuged for 5 min at 12,000 g at 4°C. The upper clear, aqueous nucleic acid phase was transferred into a new tube. 250 µl of isopropanol (Th. Geyer) was added to each sample. For RNA precipitation tubes were kept overnight at - 20°C. Then, tubes were centrifuged for 15 min at 4°C of 12,000 g and supernatant was discarded. The pellet was washed twice with 75% ethanol (Th. Geyer) in DEPC water (Carl Roth) and centrifuged for 10 min at 4 °C at 12,000 g. After the last washing step all ethanol had to evaporate by first discarding the ethanol and afterwards letting air-dry the pellet for about 30 min. The pellet was resuspended in 20 µl DEPC-treated H₂O and shook at 400 rpm at 37 °C. To prevent DNA contamination the DNAase I Amplification grade kit (Sigma-Aldrich) was used.

For each sample, 30 µl of RNA solution (60 ng/µl) in ddH₂O was sent to the High Throughput Sequencing (seq) Unit of the Genomics & Proteomics Core Facility at the DKFZ (Heidelberg, Germany) to be processed for RNA Bulk Sequencing. Samples were run through in-house quality control and only samples with an RNA integrity number (RIN) ≥ 6.0 were used for cDNA library preparation according to the TruSeq Stranded protocol (Illumina). Libraries were sequenced to 50 bp single reads on one lane on the Illumina HiSeq 4K platform. The DKFZ Omics IT and Data Management Core Facility performed the RNAseq processing workflow. Total counts per feature were imported to (⁹⁴R Core Team. R: A Language and Environment for Statistical Computing. https://www.r-project.org/. Published online 2020) and analyzed using DESeq2 (⁹⁵Love MI, Huber W, Anders S. Moderated estimation of fold change and dispersion for RNA-seq data with DESeq2. Genome Biol. 2014;15(12):550. doi:10.1186/s13059-014-0550-8). All features without any counts were removed, for differential testing with DESeq2 the formula "∼∼Batch+ Condition" was used. Features with an adjusted p-value < 0.05 for a respective comparison were used to perform gene ontology (GO) enrichment analysis using enrichGO from the clusterProfiler package (⁹⁶Yu G, Wang LG, Han Y, He QY. clusterProfiler: an R package for comparing biological themes among gene clusters. OMICS. 2012;16(5):284-287. doi:10.1089/omi.2011.0118). The organism database used for this analysis was org.Hs.eg.db (Carlson M., org.Hs.eg.db. Genome wide annotation for Human. Published online 2020). Heatmaps for RNAseq expression data show z-scaled DESeq2 normalized counts. RNA bulk seq data analysis and graphic visualizations were performed in collaboration with Dr. Anne Hoffrichter (Hector Institute for Translational Brain Research, Central Institute of Mental Health, Mannheim, Germany). Biological batch replicates of each samples ensured intra-cell lines stability of results.

### Protein isolation and measurement of protein concentration

Cells were washed with ice cold DPBS, harvested by using a cell lifter and were transferred into a 1.5 ml tube on ice. In the following the cells were centrifuged for 5 min at 5000 g at 4 °C. The supernatant was removed and the sample was stored at - 20°C. For protein isolation, cells were resuspended in 150 µl of protein lysis buffer (50 mM Tris-HCL (pH 7.4), 150 mM NaCl, 25 mM EDTA, 1% (v/v) SDS with one protease and one phosphatase inhibitor mini tablet per 10 ml of protein solution) and incubated for 10 min at room temperature and then incubated 1 hr on ice. To shear gDNA and reduce viscosity samples were sonicated (20% duty cycles, 50% output, five pulses) from a Branson Ultrasonics^{™} sonifier 250 (Thermo Fisher Scientific). Samples were centrifuged for 5 min for 5000 g at 4°C. Supernatant was transferred to a new tube. Protein concentration was measured with the BCA protein assay (Thermo Fisher Scientific) following the manufacturer's instructions. Samples were diluted 1:5 in ddH₂O and the absorption at 562 nm was measured in a PowerWave^{™} XS microplate reader (BioTek). The protein concentration of samples was calculated based on the included BSA standard dilution series.

### SDS-polyacrylamide gel electrophoresis and western immunoblotting

15 - 30 µg of protein was mixed with 6x denaturing protein sample buffer and boiled for 5 min at 95 °C. For the separation of proteins an SDS-PAGE (Bio-Rad Laboratories) was performed in a Mini-PROTEAN 2-D electrophoresis cell chamber and with the PowerPac^{™} Basic Power Supply. First, the polyacrylamide gel runs for about 30 min with 30 V for stacking of proteins and then for about 1.5 - 2 hrs at 110 V. A semi-dry blotting was performed using the Trans-Blot Turbo^{™} transfer system (Bio-Rad Laboratories) performed with 20 V and 1 A for 30 - 60 min. For blotting 0.45 µm pore size nitrocellulose blotting membrane (Sigma-Aldrich) was used. The membrane was surrounded from both sides by 6 layers of WB filter tissue (VWR). Filter tissue and membrane were wetted in transfer buffer (10% (v/v) Tris-glycine buffer, 20% (v/v) methanol, 0.08% (v/v) SDS). Membranes were blocked for 60 min in 5% BSA (Sigma-Aldrich) in TBS-T (10% (v/v) of TBS with 248 mM Tris-HCl (pH 7.4), 1.37 MNaCl, 26.8 mM KCl plus 0.1% (v/v) Tween^{®}20 (Sigma-Aldrich)) in a 50 ml tube. Primary antibodies (1:2000 Annexin A2 (rb, clone D11G2, Cell Signaling Technologies); 1:400 S100A10 (ms, clone, 4E7E10, Cell Signaling Technologies) were diluted in 5% BSA in TBS-T and were incubated over night at 4°C or for 1 h at room temperature on a rolling mixer. The membrane was washed three times in TBS-T for 10 min at room temperature on the next day. 1:15,000 infrared DyLight^{™} IR-conjugated secondary antibodies (Anti-rb 680; anti-ms 800, Cell Signaling Technologies) in TBS-T were applied for 1 hr at room temperature. Washing in TBS-T was carried out three times and once in TBS for 10 min at room temperature. Visualization of tagged proteins was carried out with Odyssey IR WB imaging system (Li-COR). Signals were normalized by 1: 20,000 β-actin levels (ms, clone 8H10D10 or rb, clone 13E5; Cell Signaling Technologies).

### Immunocytochemistry

After a first washing with PBS, cells were fixed in 4% PFA (Sigma-Aldrich), washed three times with PBS and blocked and/or permeabilized in blocking solution containing 10% FBS (Thermo Fisher Scientific) in PBS. For permeabilization 0.1% (v/v) saponin (Fluka) was added for one hr. Dilution of primary and secondary antibodies were performed in aforementioned blocking solution. Primary antibodies (Annexin A2 (rb, clone D11G2, Cell Signaling Technologies; S100A10 (ms, 4E7E10, Cell Signaling Technologies) were incubated over night at 4°C. Samples were washed three times with corresponding blocking solution. Secondary antibodies, conjugated to Alexa Fluor 488, 568 or 647 (Thermo Fisher Scientific), were diluted 1:1000 and were applied for 1 hr at room temperature. Samples were then washed once in PBS to remove unbound antibodies. Counterstaining of cell nuclei was carried out by using 300 nM DAPI (Biolegend), incubated for 5 min at room temperature and washed three times in PBS and once with ddH₂O. Slides were mounted with mounting solution (100 mM Tris-HCl (pH 8.5), 25% glycerol, 10% mowiol (Carl Roth), 0.6% DABCO^{®} (Carl Roth) on glass coverslip and air-dried overnight.

### Western Blot

For quantification region of interests (ROIs) were set around lanes and intensity was measured with the densitometry analysis in ImageJ (National Institutes of Health (NIH), open source).

### Analysis of immunofluorescence

Leica confocal TCS SP5 II microscope was used. A polygon selection of ROIs was chosen. The protein co-localization of particles was measured with the ComDet v.0.5.3 plug-in for ImageJ. A particle size (depending on experimenters' evaluation), constant for overall experiments (Annexin A2: 1.0; S100A10: 1.0) and an intensity threshold (in SD), adjusted within each experiment, was defined. For co-localization analysis the channels of proteins that have to be co-localized were merged in advance. As maximal distance between co-localized spots 4.00 pixels were stated. The number of particles was divided by the total length of the neurite in µm measured with the straight-line tool spanning the polygonal ROI.

### Statistical analysis

For statistical analysis the RStudio for macOS (Version 1.4.1106^{©} 2009) was used. Graphs were generated with RStudio and show violin blots including single data points (scatterplot) with mean ± standard deviation (SD) and boxplot with median. The Kolmogorov-Smirnov test for equality of a probability distribution and the Levene test for homogeneity of variance were calculated prior statistical analysis. In case the data does not meet the assumption for parametric testing Kruskal-Wallis test for more than two group comparisons (post hoc Wilcoxon rank sum test, *p*-value adjustment Bonferroni correction), or for a two group comparison a Mann-Whitney-U-test for independent samples was calculated. Significance levels against the respective controls were not significant (n.s) if *p* > 0.05, or significant ^{∗} *p* < 0.05. Individual figure legends contain detailed information regarding the number of replicates, sample size and the applied statistical tests.

### Plasminogen, tPA and/or plasmin affect BDNF processing in human brain cells

The inventors used human neurons differentiated from induced pluripotent stem cells (iPSCs) to investigate the extent to which the processing of pro-BDNF to m-BDNF is affected by different components of the plasminogen-plasmin cascade. These neuronal cells represent a model of the human brain. The measurements were taken in the supernatant, a model for the extracellular milieu of the human brain. When human neurons are exposed to pro-BDNF alone, practically no conversion of pro-BDNF to m-BDNF can be detected. After the addition of plasminogen, a clear band is found at the level of m-BDNF, which indicates that plasminogen has been converted by the cells into plasmin, which in turn converts pro-BDNF into m-BDNF (Figure 2A, B). Therefore, since plasminogen is the substrate in this reaction, the reaction is directly dependent on the plasminogen concentration. Additional administration of tPA shows a further significant increase in the conversion of pro-BDNF to m-BDNF in the supernatant (about 5.5-fold over plasminogen alone), which indicates that tPA is also only available to a limited extent and the processing of pro-BDNF can be further enhanced into m-BDNF by a combination of plasminogen and tPA. The conversion of pro-BDNF to m-BDNF in the combination therapy corresponds to the direct addition of plasmin. This shows that plasmin alone or in combination with plasminogen and/or tPA can also be used therapeutically (Figure 2B).

### 5-HT2A receptor agonists act via activation of the AIIt complex and processing of BDNF

In RNA bulk sequencing studies on human induced pluripotent stem cell-derived neurons, the inventors found that stimulation of human neurons with the 5-HT2A receptor agonist psilocin (as the psychoactive component of psilocybin) leads to a significant approximately 5-fold upregulation of biological processes involved in are involved in the negative regulation of blood coagulation (Figure 3A). This effect persisted even three days after psilocin administration. The biological processes mentioned in this experiment were upregulated by a factor of 10. At the same time, biological processes such as "plasminogen activation" and "fibrinolysis" were upregulated 30-fold (Figure 3B). A closer look at the genes that play a role in these processes showed that psilocin not only upregulated the core components of the AIIt complex (annexin A2 and p11), but also the plasminogen-cleaving enzyme tPA (PLAT gene, factor: 0.9 log2 fold change) and uPA (urokinase-type plasminogen activator, PLAU gene, factor: 2 log2 fold change) (Figure 3C).

*ANXA2,* the gene encoding for annexin 2, was significantly upregulated by a factor of 1.5 (log2 fold change). The S100A10 gene was significantly increased by a factor of 1.9 (log2 fold change). Furthermore, the genes of the receptors of tPA (PLGRKT, factor: 0.9 log2 fold change) and uPA (PLAUR, factor: 1.4 log2 fold change) were significantly upregulated. This is of particular interest since the plasmin level is significantly positively correlated with the number of plasminogen receptors⁴.

Also at the protein level, there was an approximately 40% increase in annexin A2 and an approximately 40% increase in P11 after psilocin administration {Annexin A2 (*M*_{Ctrl} = 1.14, *SD* = 0.38 on *M*_{Psi} = 1.63, *SD* = 0.51); P11 (*M*_{Ctrl} = 1.53, *SD* = 0.7 on *M*_{Psi} = 2.16, *SD* = 0.95)} (Figure 3D-E). In addition, the co-localization of annexin A2 and P11 was elevated (from *M*_{Ctrl} = 0.11, *SD* = 0.09 to *M*_{3d} = 0.15, *SD* = 0.12), pointing towards an increased formation of the complex (Figure 3F-G). These results indicate that 5-HT2A receptor agonists produce an increase in the capacity of the central nervous enzyme cascade to process pro-BDNF into m-BDNF. This suggests a direct link to the effect of 5-HT2A receptor agonists in psychiatric disorders.

### Plasminogen, tPA and/or plasmin synergistically potentiate the effect of 5-HT2A receptor agonist in BDNF processing

In a cell culture model, the inventors investigated the processing of pro-BDNF into m-BDNF in cells treated with the 5-HT2A receptor agonist psilocin for three days and in untreated controls. In the experiment, plasminogen or plasminogen + tPA was added. The previous treatment with 5-HT2A receptor agonists was able to increase the processing of pro-BDNF to m-BDNF within each condition by a factor of about 2-3 (Figure 4 A, B, D). At the same time, there is a decrease in pro-BDNF ((Figure 4 B, C, D). BDNF processing can also be enhanced with TCB-2, another 5-HT2A receptor agonist. This shows that 5-HT2A receptor agonists and components of the Plasminogen-plasmin cascade act synergistically on the processing of pro-BDNF, since the enzymatic machinery is now up-regulated in addition to the substrates.

In conclusion, the inventor's results show that the bioavailability of plasminogen, tPA and plasmin can be manipulated via external application. Both plasminogen and tPA are approved for therapeutic applications in human medicine. Plasmin is in clinical testing, and thus their effectiveness and tolerability in humans have already been tested for the approved therapeutic applications.

### References

1. Kowiański P, Lietzau G, Czuba E, Waśkow M, Steliga A, Moryś J. BDNF: A Key Factor with Multipotent Impact on Brain Signaling and Synaptic Plasticity. *Cell Mol Neurobiol.* 2018;38(3):579-593. doi:10.1007/s10571-017-0510-4
2. Lu B, Pang PT, Woo NH. The yin and yang of neurotrophin action. Nat Rev Neurosci. 2005;6(8):603-614. doi:10.1038/nrn1726
3. Pang PT, Teng HK, Zaitsev E, et al. Cleavage of proBDNF by tPA/plasmin is essential for long-term hippocampal plasticity. Science. 2004;306(5695):487-491. doi: 1 0.1 1 26/science. 1 1 00135
4. Bharadwaj A, Bydoun M, Holloway R, Waisman D. Annexin A2 heterotetramer: Structure and function. Int J Mol Sci. 2013;14(3):6259-6305. doi:10.3390/ijms14036259
5. Jiang H, Chen S, Li C, et al. The serum protein levels of the tPA-BDNF pathway are implicated in depression and antidepressant treatment. Transl Psychiatry. 2017;7(4):e1079-e1079. doi:10.1038/tp.2017.43
6. Wang M, Xie Y, Qin D. Proteolytic cleavage of proBDNF to mBDNF in neuropsychiatric and neurodegenerative diseases. Brain Res Bull. 2021;166:172-184. doi:10.1016/j.brainresbull.2020.11.005
7. Zhou L, Xiong J, Lim Y, et al. Upregulation of blood proBDNF and its receptors in major depression. J Affect Disord. 2013;150(3):776-784. doi:10.1016/j.jad.2013.03.002
8. Ludka FK, Cunha MP, Dal-Cim T, et al. Atorvastatin Protects from Aβ1-40-Induced Cell Damage and Depressive-Like Behavior via ProBDNF Cleavage. Mol Neurobiol. 2017;54(8):6163-6173. doi:10.1007/s12035-016-0134-6
9. Li J, Chen J, Ma N, et al. Effects of corticosterone on the expression of mature brain-derived neurotrophic factor (mBDNF) and proBDNF in the hippocampal dentate gyrus. Behavioural Brain Research. 2019;365:150-156. doi:10.1016/j.bbr.2019.03.010
10. Yang CR, Zhang XY, Liu Y, et al. Antidepressant Drugs Correct the Imbalance Between proBDNF/p75NTR/Sortilin and Mature BDNF/TrkB in the Brain of Mice with Chronic Stress. Neurotox Res. 2020;37(1): 171-182. doi:10.1007/s12640-019-00101-2
11. Lin LY, Luo SY, Al-Hawwas M, Herselman MF, Zhou XF, Bobrovskaya L. The Long-Term Effects of Ethanol and Corticosterone on the Mood-Related Behaviours and the Balance Between Mature BDNF and proBDNF in Mice. Journal of Molecular Neuroscience. 2019;69(1):60-68. doi:10.1007/s12031-019-01328-6
12. Qiao H, An SC, Xu C, Ma XM. Role of proBDNF and BDNF in dendritic spine plasticity and depressive-like behaviors induced by an animal model of depression. Brain Res. 2017;1663:29-37. doi:10.1016j.brainres.2017.02.020
13. Svenningsson P, Kim Y, Warner-Schmidt J, Oh YS, Greengard P. P11 and its role in depression and therapeutic responses to antidepressants. Nat Rev Neurosci. 2013;14(10):673-680. doi:10.1038/nrn3564
14. Idell RD, Florova G, Komissarov AA, Shetty S, Girard RBS, Idell S. The fibrinolytic system: A new target for treatment of depression with psychedelics. Med Hypotheses. 2017;100:46-53. doi:10.1016/j.mehy.2017.01.013
15. Alboni S, van Dijk RM, Poggini S, et al. Fluoxetine effects on molecular, cellular and behavioral endophenotypes of depression are driven by the living environment. Mol Psychiatry. 2017;22(4):552-561. doi:10.1038/mp.2015.142
16. Gueorguieva R. Trajectories of Depression Severity in Clinical Trials of Duloxetine. Arch Gen Psychiatry. 2011;68(12): 1227. doi:10.1001/archgenpsychiatry.2011.132
17. Machado-Vieira R, Baumann J, Wheeler-Castillo C, et al. The Timing of Antidepressant Effects: A Comparison of Diverse Pharmacological and Somatic Treatments. Pharmaceuticals. 2010;3(1): 19-41. doi:10.3390/ph3010019
18. Baldessarini RJ, Tondo L, Ghiani C, Lepri B. Illness Risk Following Rapid Versus Gradual Discontinuation of Antidepressants. American Journal of Psychiatry. 2010;167(8):934-941. doi:10.1176/appi.ajp.2010.09060880
19. Faedda GL. Outcome After Rapid vs Gradual Discontinuation of Lithium Treatment in Bipolar Disorders. Arch Gen Psychiatry. 1993;50(6):448. doi:10.1001/archpsyc.1993.01820180046005
20. Viguera AC. Clinical Risk Following Abrupt and Gradual Withdrawal of Maintenance Neuroleptic Treatment. Arch Gen Psychiatry. 1997;54(1):49. doi:10.1001/archpsyc.1997.01830130055011
21. Fava GA. Do Antidepressant and Antianxiety Drugs Increase Chronicity in Affective Disorders? Psychother Psychosom. 1994;61(3-4):125-131. doi:10.1159/000288880
22. Young AM, Goudie AJ. Adaptive processes regulating tolerance to behavioral effects of drugs. In: Bloom FE, Kupfer DJ, eds. Psychopharmacology: The Fourth Generation of Progress. Raven Press; 1995:733-742.
23. Byrne SE, Rothschild AJ. Loss of Antidepressant Efficacy During Maintenance Therapy. J Clin Psychiatry. 1998;59(6):279-288. doi: 1 0.4088/JCP .v59n0602
24. Thase ME, Rush JA. Treatment-resistant depression. In: Psychopharmacology. Raven; 1995.
25. El-Mallakh RS, Gao Y, Jeannie Roberts R. Tardive dysphoria: The role of long term antidepressant use in-inducing chronic depression. Med Hypotheses. 2011;76(6):769-773. doi:10.1016/j.mehy.2011.01.020
26. Fava GA, Offidani E. The mechanisms of tolerance in antidepressant action. Prog Neuropsychopharmacol Biol Psychiatry. 2011;35(7):1593-1602. doi:10.1016/j.pnpbp.2010.07.026
27. Miller G. Is pharma running out of brainy ideas? Science. 2010;329(5991):502-504. doi:10.1126/science.329.5991.502
28. Schenberg EE. Psychedelic-Assisted Psychotherapy: A Paradigm Shift in Psychiatric Research and Development. Front Pharmacol. 2018;9. doi:10.3389/fphar.2018.00733
29. Catalá-López F, Gènova-Maleras R, Vieta E, Tabarés-Seisdedos R. The increasing burden of mental and neurological disorders. Eur Neuropsychopharmacol. 2013;23(11): 1337-1339. doi:10.1016/j.euroneuro.2013.04.001
30. Autry AE, Monteggia LM. Brain-derived neurotrophic factor and neuropsychiatric disorders. Pharmacol Rev. 2012;64(2). doi:10.1124/pr.111.005108
31. Nibuya M, Morinobu S, Duman R. Regulation of BDNF and trkB mRNA in rat brain by chronic electroconvulsive seizure and antidepressant drug treatments. The Journal of Neuroscience. 1995;15(11):7539-7547. doi:10.1523/JNEUROSCI.15-11-07539.1995
32. Segawa M, Morinobu S, Matsumoto T, Fuchikami M, Yamawaki S. Electroconvulsive seizure, but not imipramine, rapidly up-regulates pro-BDNF and t-PA, leading to mature BDNF production, in the rat hippocampus. Int JNeuropsychopharmacol. 2013;16(2):339-350. doi:10.1017/S1461145712000053
33. Sen S, Duman R, Sanacora G. Serum Brain-Derived Neurotrophic Factor, Depression, and Antidepressant Medications: Meta-Analyses and Implications. Biol Psychiatry. 2008;64(6):527-532. doi:10.1016/j.biopsych.2008.05.005
34. Svenningsson P, Chergui K, Rachleff I, et al. Alterations in 5-HT1B receptor function by p11 in depression-like states. Science. 2006;311(5757):77-80. doi:10.1126/science.1117571
35. Tramontina JF, Andreazza AC, Kauer-Sant'anna M, et al. Brain-derived neurotrophic factor serum levels before and after treatment for acute mania. Neurosci Lett. 2009;452(2):111-113. doi: 10.1 016/j .neulet.2009.01.028
36. Castrén E, Rantamäki T. The role of BDNF and its receptors in depression and antidepressant drug action: Reactivation of developmental plasticity. Dev Neurobiol. 2010;70(5):289-297. doi:10.1002/dneu.20758
37. Duman RS, Monteggia LM. A Neurotrophic Model for Stress-Related Mood Disorders. Biol Psychiatry. 2006;59(12):1116-1127. doi:10.1016/j.biopsych.2006.02.013
38. Castren E, Vikar V, Rantamaki T. Role of neurotrophic factors in depression. Curr Opin Pharmacol. 2007;7(1):18-21. doi:10.1016/j.coph.2006.08.009
39. Polyakova M, Stuke K, Schuemberg K, Mueller K, Schoenknecht P, Schroeter ML. BDNF as a biomarker for successful treatment of mood disorders: a systematic & quantitative meta-analysis. J Affect Disord. 2015;174:432-440. doi:10.1016/j.jad.2014.11.044
40. Tang M, Jiang P, Li H, et al. Antidepressant-like effect of n-3 PUFAs in CUMS rats: role of tPA/PAI-1 system. Physiol Behav. 2015;139:210-215. doi:10.1016/j.physbeh.2014.11.054
41. Sartori CR, Vieira AS, Ferrari EM, Langone F, Tongiorgi E, Parada CA. The antidepressive effect of the physical exercise correlates with increased levels of mature BDNF, and proBDNF proteolytic cleavage-related genes, p11 and tPA. Neuroscience. 2011;180:9-18. doi:10.1016/j.neuroscience.2011.02.055
42. Ding Q, Ying Z, Gómez-Pinilla F. Exercise influences hippocampal plasticity by modulating brain-derived neurotrophic factor processing. Neuroscience. 2011;192:773-780. doi:10.1016/j.neuroscience.2011.06.032
43. Bahi A, Dreyer JL. Hippocampus-specific deletion of tissue plasminogen activator "tPA" in adult mice impairs depression- and anxiety-like behaviors. European Neuropsychopharmacology. 2012;22(9):672-682. doi:10.1016/j.euroneuro.2012.01.008
44. Benaud C, Gentil BJ, Assard N, et al. AHNAK interaction with the annexin 2/S100A10 complex regulates cell membrane cytoarchitecture. Journal of Cell Biology. 2004; 164(1): 133-144. doi:10.1083/jcb.200307098
45. Burford NT, Traynor JR, Alt A. Positive allosteric modulators of the µ-opioid receptor: a novel approach for future pain medications. Br J Pharmacol. 2015;172(2):277-286. doi:10.1111/bph.12599
46. Collen D, Topol EJ, Tiefenbrunn AJ, et al. Coronary thrombolysis with recombinant human tissue-type plasminogen activator: a prospective, randomized, placebo-controlled trial. Circulation. 1984;70(6):1012-1017. doi:10.1161/01.cir.70.6.1012
47. TIMI Study Group. The Thrombolysis in Myocardial Infarction (TIMI) trial. Phase I findings. N Engl J Med. 1985;312(14):932-936. doi:10.1056/NEJM198504043121437
48. Dalen JE, Gore JM, Braunwald E, et al. Six- and twelve-month follow-up of the phase I Thrombolysis in Myocardial Infarction (TIMI) trial. Am J Cardiol. 1988;62(4):179-185. doi:10.1016/0002-9149(88)90208-1
49. National Institute of Neurological Disorders and Stroke rt-PA Stroke Study Group. Tissue Plasminogen Activator for Acute Ischemic Stroke. New England Journal of Medicine. 1995;333(24):1581-1588. doi:10.1056/NEJM199512143332401
50. Kwiatkowski TG, Libman RB, Frankel M, et al. Effects of Tissue Plasminogen Activator for Acute Ischemic Stroke at One Year. New England Journal of Medicine. 1999;340(23):1781-1787. doi:10.1056/NEJM199906103402302
51. Pennica D, Holmes WE, Kohr WJ, et al. Cloning and expression of human tissue-type plasminogen activator cDNA in E. coli. Nature. 1983;301(5897):214-221. doi:10.1038/301214a0
52. Liu Z, Xiong Y, Chopp M. Intranasal tPA Application for Axonal Remodeling in Rodent Stroke and Traumatic Brain Injury Models. In: ; 2019:101-115. doi:10.1007/978-3-030-16715-8_9
53. Liu Z, Li Y, Zhang L, et al. Subacute intranasal administration of tissue plasminogen activator increases functional recovery and axonal remodeling after stroke in rats. Neurobiol Dis. 2012;45(2):804-809. doi:10.1016/j.nbd.2011.11.004
54. Fan M, Xu H, Wang L, et al. Tissue Plasminogen Activator Neurotoxicity is Neutralized by Recombinant ADAMTS 13. Sci Rep. 2016;6(1):25971. doi:10.1038/srep25971
55. Medcalf RL. Plasminogen and stroke: more is better. Journal of Thrombosis and Haemostasis. 2016; 14(9):1819-1821. doi:10.1111/jth.13399
56. Marder VJ, Landskroner K, Novokhatny V, et al. Plasmin induces local thrombolysis without causing hemorrhage: a comparison with tissue plasminogen activator in the rabbit. Thromb Haemost. 2001;86(3):739-745.
57. Marder VJ, Jahan R, Gruber T, Goyal A, Arora V. Thrombolysis with plasmin: implications for stroke treatment. Stroke. 2010;41(10 Suppl):S45-9. doi:10.1161/STROKEAHA.110.595157
58. Qian Z, Gilbert ME, Colicos MA, Kandel ER, Kuhl D. Tissue-plasminogen activator is induced as an immediate-early gene during seizure, kindling and long-term potentiation. Nature. 1993;361(6411):453-457. doi:10.1038/361453a0
59. Huang YY, Bach ME, Lipp HP, et al. Mice lacking the gene encoding tissue-type plasminogen activator show a selective interference with late-phase long-term potentiation in both Schaffer collateral and mossy fiber pathways. Proceedings of the National Academy of Sciences. 1996;93(16):8699-8704. doi:10.1073/pnas.93.16.8699
60. Madani R. Enhanced hippocampal long-term potentiation and learning by increased neuronal expression of tissue-type plasminogen activator in transgenic mice. EMBO J. 1999;18(11):3007-3012. doi:10.1093/emboj/18.11.3007
61. Baranes D, Lederfein D, Huang YY, Chen M, Bailey CH, Kandel ER. Tissue Plasminogen Activator Contributes to the Late Phase of LTP and to Synaptic Growth in the Hippocampal Mossy Fiber Pathway. Neuron. 1998;21(4):813-825. doi:10.1016/S0896-6273(00)80597-8
62. Fernández-Monreal M, López-Atalaya JP, Benchenane K, et al. Is tissue-type plasminogen activator a neuromodulator? Molecular and Cellular Neuroscience. 2004;25(4):594-601. doi:10.1016/j.mcn.2003.11.002
63. Chen ZL, Strickland S. Neuronal Death in the Hippocampus Is Promoted by Plasmin-Catalyzed Degradation of Laminin. Cell. 1997;91(7):917-925. doi:10.1016/S0092-8674(00)80483-3
64. Hu K, Yang J, Tanaka S, Gonias SL, Mars WM, Liu Y. Tissue-type Plasminogen Activator Acts as a Cytokine That Triggers Intracellular Signal Transduction and Induces Matrix Metalloproteinase-9 Gene Expression. Journal of Biological Chemistry. 2006;281(4):2120-2127. doi:10.1074/jbc.M504988200
65. Wu YP, Siao CJ, Lu W, et al. The Tissue Plasminogen Activator (Tpa/Plasmin) Extracellular Proteolytic System Regulates Seizure-Induced Hippocampal Mossy Fiber Outgrowth through a Proteoglycan Substrate. Journal of Cell Biology. 2000;148(6):1295-1304. doi:10.1083/jcb.148.6.1295
66. Samson AL, Medcalf RL. Tissue-Type Plasminogen Activator: A Multifaceted Modulator of Neurotransmission and Synaptic Plasticity. Neuron. 2006;50(5):673-678. doi:10.1016/j.neuron.2006.04.013
67. Anisman H, Du L, Palkovits M, et al. Serotonin receptor subtype and p11 mRNA expression in stress-relevant brain regions of suicide and control subjects. J Psychiatry Neurosci. 2008;33(2):131-141.
68. Warner-Schmidt JL, Chen EY, Zhang X, et al. A role for p11 in the antidepressant action of brain-derived neurotrophic factor. Biol Psychiatry. 2010;68(6):528-535. doi:10.1016/j.biopsych.2010.04.029
69. Egeland M, Warner-Schmidt J, Greengard P, Svenningsson P. Neurogenic Effects of Fluoxetine Are Attenuated in p11 (S100A10) Knockout Mice. Biol Psychiatry. 2010;67(11):1048-1056. doi:10.1016/j.biopsych.2010.01.024
70. Nichols D, Johnson M, Nichols C. Psychedelics as Medicines: An Emerging New Paradigm. Clin Pharmacol Ther. 2017;101(2):209-219. doi:10.1002/cpt.557
71. Carhart-Harris RL, Goodwin GM. The Therapeutic Potential of Psychedelic Drugs: Past, Present, and Future. Neuropsychopharmacology. 2017;42(11):2105-2113. doi:10.1038/npp.2017.84
72. Corriger A, Pickering G. Ketamine and depression: a narrative review. Drug Des Devel Ther. 2019;Volume 13:3051-3067. doi:10.2147/DDDT.S221437
73. Lowe H, Toyang N, Steele B, et al. The Therapeutic Potential of Psilocybin. Molecules. 2021;26(10):2948. doi:10.3390/molecules26102948
74. Ly C, Greb AC, Cameron LP, et al. Psychedelics Promote Structural and Functional Neural Plasticity. Cell Rep. 2018;23(11):3170-3182. doi:10.1016/j.celrep.2018.05.022
75. de Vos CMH, Mason NL, Kuypers KPC. Psychedelics and Neuroplasticity: A Systematic Review Unraveling the Biological Underpinnings of Psychedelics. Front Psychiatry. 2021;12:724606. doi:10.3389/fpsyt.2021.724606
76. Jefsen OH, Elfving B, Wegener G, Müller HK. Transcriptional regulation in the rat prefrontal cortex and hippocampus after a single administration of psilocybin. J Psychopharmacol. 2021;35(4). doi: 10.1177/0269881120959614
77. Martin DA, Marona-Lewicka D, Nichols DE, Nichols CD. Chronic LSD alters gene expression profiles in the mPFC relevant to schizophrenia. Neuropharmacology. 2014;83:1-8. doi:10.1016/j.neuropharm.2014.03.013
78. Vaidya VA, Marek GJ, Aghajanian GK, Duman RS. 5-HT2A receptor-mediated regulation of brain-derived neurotrophic factor mRNA in the hippocampus and the neocortex. J Neurosci. 1997;17(8).
79. Zhang F, Luo J, Zhu X. Ketamine ameliorates depressive-like behaviors by tPA-mediated conversion of proBDNF to mBDNF in the hippocampus of stressed rats. Psychiatry Res. 2018;269:646-651. doi:10.1016/j.psychres.2018.08.075
80. Holze F, Vizeli P, Ley L, et al. Acute dose-dependent effects of lysergic acid diethylamide in a double-blind placebo-controlled study in healthy subjects. Neuropsychopharmacology. 2021;46(3):537-544. doi:10.1038/s41386-020-00883-6
81. Hutten NRPW, Mason NL, Dolder PC, et al. Low Doses of LSD Acutely Increase BDNF Blood Plasma Levels in Healthy Volunteers. ACS Pharmacol Transl Sci. 2021;4(2):461-466. doi:10.1021/acsptsci.0c00099
82. Fortunato JJ, Réus GZ, Kirsch TR, et al. Acute harmine administration induces antidepressive-like effects and increases BDNF levels in the rat hippocampus. Prog Neuropsychopharmacol Biol Psychiatry. 2009;33(8). doi:10.1016/j.pnpbp.2009.07.021
83. Liu F, Wu J, Gong Y, et al. Harmine produces antidepressant-like effects via restoration of astrocytic functions. Prog Neuropsychopharmacol Biol Psychiatry. 2017;79(Pt B). doi:10.1016/j.pnpbp.2017.06.012
84. Colaço CS, Alves SS, Nolli LM, et al. Toxicity of ayahuasca after 28 days daily exposure and effects on monoamines and brain-derived neurotrophic factor (BDNF) in brain of Wistar rats. Metab Brain Dis. 2020;35(5):739-751. doi:10.1007/s11011-020-00547-w
85. Nardai S, László M, Szabó A, et al. N,N-dimethyltryptamine reduces infarct size and improves functional recovery following transient focal brain ischemia in rats. Exp Neurol. 2020;327:113245. doi:10.1016/j.expneurol.2020.113245
86. Veldman ER, Mamula D, Jiang H, et al. P11 (S100A10) as a potential predictor of ketamine response in patients with SSRI-resistant depression. J Affect Disord. 2021;290:240-244. doi:10.1016/j.jad.2021.04.055
87. Sun HL, Zhou ZQ, Zhang GF, et al. Role of hippocampal p11 in the sustained antidepressant effect of ketamine in the chronic unpredictable mild stress model. Transl Psychiatry. 2016;6(2):e741-e741. doi:10.1038/tp.2016.21
88. Nichols CD, Sanders-Bush E. A single dose of lysergic acid diethylamide influences gene expression patterns within the mammalian brain. Neuropsychopharmacology. 2002;26(5). doi:10.1016/S0893-133X(01)00405-5
89. Galvão-Coelho NL, de Menezes Galvão AC, de Almeida RN, et al. Changes in inflammatory biomarkers are related to the antidepressant effects of Ayahuasca. Journal of Psychopharmacology. 2020;34(10):1125-1133. doi:10.1177/0269881120936486
90. Almeida RN de, Galvão AC de M, da Silva FS, et al. Modulation of Serum Brain-Derived Neurotrophic Factor by a Single Dose of Ayahuasca: Observation From a Randomized Controlled Trial. Front Psychol. 2019;10. doi:10.3389/fpsyg.2019.01234
91. Yamada Y. Nucleic Acid Drugs-Current Status, Issues, and Expectations for Exosomes. Cancers (Basel). 2021;13(19):5002. doi:10.3390/cancers13195002
92. Kulkarni JA, Witzigmann D, Thomson SB, et al. The current landscape of nucleic acid therapeutics. Nat Nanotechnol. 2021;16(6):630-643. doi:10.1038/s41565-021-00898-0
93. Morrow CS, Porter TJ, Xu N, et al. Vimentin Coordinates Protein Turnover at the Aggresome during Neural Stem Cell Quiescence Exit. Cell Stem Cell. 2020;26(4):558-568.e9. doi:10.1016/j.stem.2020.01.018
94. R Core Team. R: A Language and Environment for Statistical Computing. https://www.r-project.org/. Published online 2020.
95. Love MI, Huber W, Anders S. Moderated estimation of fold change and dispersion for RNA-seq data with DESeq2. Genome Biol. 2014;15(12):550. doi:10.1186/s13059-014-0550-8
96. Yu G, Wang LG, Han Y, He QY. clusterProfiler: an R package for comparing biological themes among gene clusters. OMICS. 2012;16(5):284-287. doi:10.1089/omi.2011.0118

## Claims

1. At least one of a protein selected from group consisting of tPA, uPA, plasminogen, plasmin, and functional fragments thereof, or a nucleic acid encoding said tPA, uPA, plasminogen, plasmin, and functional fragments thereof for use in the prevention or treatment of a psychiatric disorder or disease in a subject, wherein preferably the protein tPA, uPA, and plasminogen or functional fragments thereof, or the nucleic acid encoding said tPA, uPA, and plasminogen or functional fragments thereof are used in combination.

2. The at least one protein for use according to claim 1, wherein the protein or functional fragment thereof has been recombinantly produced, or wherein the nucleic acid or functional fragment thereof is RNA, DNA, or mixtures thereof, is in the form of naked RNA, an expression cassette or vector, or has been recombinantly or synthetically produced or in vitro transcribed.

3. A method for identifying a compound that increases the expression, amount and/or biological activity of a protein of the AIIt complex and/or PLGRKT in a cell of a subject, comprising the steps of
i) contacting at least one candidate compound with a cell that expresses at least one protein of the AIIt complex and/or PLGRKT, and
ii) detecting an increase of the expression, amount and/or biological activity of the at least one protein of the AIIt complex and/or PLGRKT in the cell in the presence of the candidate compound compared to the expression, amount and/or biological activity in the absence of the candidate compound,
optionally in the presence of at least one psychoplastogenic agent, at least one 5-HT2A receptor agonist, ketamine, SSRI antidepressant, mGluR2 agonist, mGlur2/3 agonist, positive allosteric modulator (PAM), cytokine, and/or ECT,
wherein an increase of the expression, amount and/or biological activity of the at least one protein of the AIIt complex and/or PLGRKT in the cell identifies a compound that increases the expression, amount and/or biological activity of a protein of the AIIt complex and/or PLGRKT in a cell of a subject,
wherein preferably the at least one protein of the AIIt complex and/or PLGRKT is selected from the group consisting of S100A10, ANXA2, tPA, uPA, PAUR, PLGRKT, plasminogen, and plasmin, and/or wherein the compound specifically increases the expression, amount and/or biological activity of one protein of the AIIt complex.

4. The method according to claim 3, wherein the cell is a neuronal cell, such as, for example, a human induced pluripotent stem cell-derived neuronal cell.

5. The method according to claim 3 or 4, wherein the compound is selected from the group consisting of a natural compound, a plant extract, a peptide, a protein, a small molecule (less than about 500 Da), a nucleic acid, a DNA, an RNA, an siRNA, and an antibody or antigen binding fragment thereof.

6. A method for producing a pharmaceutical composition, comprising performing a method according to any one of claims 3 to 5, and admixing the compound as identified with at least one pharmaceutically carrier.

7. A pharmaceutical composition, comprising at least one protein or functional fragment thereof, at least one nucleic acid or functional fragment thereof, at least one expression cassette or at least one vector for use according to claim 1 or 2, together with at least one pharmaceutically acceptable carrier, or as produced according to claim 6, for use in medicine, in particular for use in the prevention or treatment of a psychiatric disorder or disease in a subject, wherein preferably the composition is for injection, oral and/or nasal application.

8. The pharmaceutical composition for use according to claim 7, wherein the use is for an improvement or amplification of the therapeutic effect of a compound selected from the group consisting of an HT2A receptor agonist, ketamine, psilocin, mGluR2 agonist, mGluR2/3 agonist, positive allosteric modulator (PAM), neurolepticum, lithium, an antidepressive agent, such as, for example, an SSRI, and a psychoplastogenic agent in the prevention or treatment of a psychiatric disorder or disease in a subject.

9. The pharmaceutical composition for use according to claim 7 or 8, wherein the use is for an improvement or amplification of the therapeutic effect of electroconvulsive therapy (ECT) and/or psychotherapy in a subject.

10. The pharmaceutical composition for use according to claim 8 or 9, wherein the use is as adjuvant therapy or in combination with the compound or therapy, such as, for example, at least one 5-HT2A receptor agonist, ketamine, SSRI antidepressant, mGluR2 agonist, mGluR2/3 agonist, positive allosteric modulator (PAM), cytokine, and/or ECT, and preferably the improvement or amplification of the therapeutic effect is synergistic.

11. The at least one protein for use according to claim 1 or 4 or the use according to any one of claims 7 to 10, wherein the psychiatric disorder or disease in the subject is selected from the group consisting of depression, treatment-resistant depression, affective disorders, comorbid depressive symptoms, substance addiction and abuse, obsessive-compulsive disorders, personality disorders (e.g., borderline), autism, Tourette syndrome, and anxiety disorders.

12. Use of at least one of a protein selected from group consisting of tPA, uPA, plasminogen, plasmin, and functional fragments thereof, or a nucleic acid encoding said tPA, uPA, plasminogen, plasmin, and functional fragments thereof for improving or amplifying the therapeutic effect of electroconvulsive therapy (ECT), psychotherapy or a compound, such as, for example, at least one 5-HT2A receptor agonist, ketamine, SSRI antidepressant, mGluR2 agonist, mGluR2/3 agonist, positive allosteric modulator (PAM) and/or cytokine, in the prevention or treatment of a psychiatric disorder or disease in a subject, wherein preferably the protein uPA, tPA and plasminogen or functional fragments thereof, or the nucleic acid encoding said uPA, tPA and plasminogen or functional fragments thereof are used in combination.
